# EUROPEAN PATENT APPLICATION

(11) **EP 1 580 266 A1**
(43) Date of publication of application: **28.09.2005**
(21) Application number: 03772678.3
(22) Date of filing: 12.11.2003
(51) Int. Cl.: C12N 15/09, C12Q 1/02, C12Q 1/68, G01N 33/15, G01N 33/50, G01N 33/53, C07K 14/47, A61K 31/7088, A61K 39/395, A61K 45/00, A61K 48/00, A61P 3/10, A61P 13/12

(54) **SCREENING METHOD**

(30) Priority: 13.11.2002 JP 2002329778
(71) Applicant: Takeda Pharmaceutical Company Limited, Osaka 541-0045 (JP)
(72) Inventor: MATSUO,Takanori c/o Takeda Pharm. Company Limited, Osaka-shi, Osaka 532-8686 (JP); TSUGE, Hiroko, c/o Takeda Pharm. Company Limited, Osaka-shi, Osaka 532-8686 (JP); HAZAMA, Masatoshi, c/o Takeda Pharm. Company Ltd., Osaka-shi, Osaka 532-8686 (JP)
(74) Representative: Lewin, John Harvey
(86) International application number: PCT/JP2003/014339
(87) International publication number: WO 2004/044199

(57) **Abstract**

The present invention provides a screening method for a prophylactic or therapeutic substance for a disease associated with a protein comprising the same or substantially the same amino acid sequence as the amino acid sequence shown by SEQ ID NO:2 or a salt thereof, for example, a renal disease or diabetes, which comprises using the protein or a partial peptide thereof or a salt thereof, or a polynucleotide that encodes the protein or a partial peptide thereof.

## Description

### Field of the Invention

The present invention relates to a novel use of Transforming Growth Factor-β Stimulated Clone-22 (hereinafter abbreviated as TSC-22), a polynucleotide that encodes the same, an antibody against the same, and the like, particularly to a screening method for a prophylactic or therapeutic agent for a renal disease and the like using TSC-22 or a polynucleotide that encodes the same.

### Background Art

At present, angiotensin-converting enzyme (ACE) inhibitors and the like are used as therapeutic agents for renal diseases but these agents are faulty in that they are not applicable to patients with severe renal dysfunction because they influence renal hemodynamics. Therefore, there is a strong demand for the development of a novel prophylactic or therapeutic agent for renal diseases that is free from such side effects.

Any renal disease is accompanied by renal fibrosis in the process to end-stage renal failure. As a factor that plays a central role in renal fibrosis, transforming growth factor-β (hereinafter abbreviated as TGF-β) can be mentioned. It has been reported that when decorin, which is an endogenous factor to neutralize TGF-β, an anti-TGF-β antibody or a TGF-β antisense oligonucleotide, is administered to a rat model of glomerulonephritis, extracellular matrix (ECM) production is remarkably inhibited and the progression of glomerulosclerosis is suppressed. It is known that introducing the TGF-β gene in vivo causes ECM accumulation and induces glomerulonephritis.

TGF-β exhibits a broad range of physiological activities by regulating the transcription of various target genes from its receptor via intracellular signal transduction factors, which are generically referred to as Smad. Therefore, the possibility exists that a molecule located downstream of the TGF-β signal transduction system is actually responsible for the progression of renal dysfunction.

TSC-22, one of the genes that respond to TGF-β stimuli, is a gene that encodes a protein having a leucine zipper (LZ) domain, for the first time isolated from a mouse osteoblastic cell line *(J. Biol. Chem. ,* 267(15): 10219-24 (1992)), and the human homologue thereof encodes a protein consisting of 144 amino acids (human-mouse (rat) homology is about 99% identity at the amino acid level; *Biochem. Biophys. Res.* Commun., 222(3): 821-6 (1996)). The TSC-22 protein has an LZ domain often found in transcription regulatory factors but lacks already known DNA-binding regions (basic amino acid regions, helix-loop-helix (HLH) motif and the like), and is therefore expected to act as a transcription suppression factor; however, it has also been reported to bind to the GC element of the type C natriuretic peptide gene to promote the transcription thereof *(Eur. J. Biochem. , 242*(3): 460-6 (1996)), and the physiological function thereof remains to be understood fully.

It is an object of the present invention to provide a novel screening method that is essential to the development of a prophylactic or therapeutic agent for renal diseases and the like, which shows superior effect and no side effects, and that is suitable for the search of a substance useful as the prophylactic or therapeutic agent.

### Summary of the Invention

With the aim of solving the above-described problems, the present inventors conducted diligent investigations, and for the first time found that the expression of renal TSC-22 in various renal disease model animals has increased remarkably in glomerular epithelial cells and tubular epithelial cells, and also for the first time found that a therapeutic effect on renal diseases is obtained by suppressing the expression of renal TSC-22 in the renal disease model animals. The inventors conducted further investigations based on these findings, and developed the present invention.

Accordingly, the present invention relates to:
[1] a screening method for a prophylactic or therapeutic substance for a disease associated with a protein comprising the same or substantially the same amino acid sequence as the amino acid sequence shown by SEQ ID NO:2 or a salt thereof, which comprises using said protein or a partial peptide thereof or a salt thereof;
[2] a screening method for a prophylactic or therapeutic substance for a disease associated with a protein comprising the amino acid sequence shown by SEQ ID NO:2 or a salt thereof, which comprises using said protein or a partial peptide thereof or a salt thereof;
[3] the screening method described in [1] above, wherein the disease is diabetes or a renal disease;
[4] the screening method described in [1] above, wherein the disease is diabetic nephropathy;
[5] the screening method described in [1] above, which comprises cultivating a cell having an ability to produce a protein comprising the same or substantially the same amino acid sequence as the amino acid sequence shown by SEQ ID NO:2 or a partial peptide thereof or a salt thereof in the presence and absence of a test substance, and comparing the amounts of said protein or a partial peptide thereof or a salt thereof produced under the two conditions;
[6] a screening kit for a prophylactic or therapeutic substance for a disease associated with a protein comprising the same or substantially the same amino acid sequence as the amino acid sequence shown by SEQ ID NO:2 or a salt thereof, which includes (a) a cell having an ability to produce said protein or a partial peptide thereof or a salt thereof, and (b) a substance selected from the group consisting of an antibody against said protein or a partial peptide thereof or a salt thereof, a polynucleotide to which said protein or a partial peptide thereof or a salt thereof can bind, and a transcription regulatory factor capable of interacting with said protein or a partial peptide thereof or a salt thereof;
[7] the screening method described in [1] above, which comprises comparing the activities of a protein comprising the same or substantially the same amino acid sequence as the amino acid sequence shown by SEQ ID NO:2 or a partial peptide thereof or a salt thereof in the presence and absence of a test substance;
[8] a screening kit for a prophylactic or therapeutic substance for a disease associated with a protein comprising the same or substantially the same amino acid sequence as the amino acid sequence shown by SEQ ID NO:2 or a salt thereof, which includes (a) said protein or a partial peptide thereof or a salt thereof, and (b) a polynucleotide to which said protein or a partial peptide thereof or a salt thereof can bind or a transcription regulatory factor capable of interacting with said protein or a partial peptide thereof or a salt thereof;
[9] the screening method described in [7] above, which comprises cultivating a cell containing a gene whose expression is controlled by a protein comprising the same or substantially the same amino acid sequence as the amino acid sequence shown by SEQ ID NO:2 or a partial peptide thereof or a salt thereof with said protein or a partial peptide thereof or a salt thereof in the presence and absence of a test substance, and comparing the expressions of said gene under the two conditions;
[10] a screening kit for a prophylactic or therapeutic substance for a disease associated with a protein comprising the same or substantially the same amino acid sequence as the amino acid sequence shown by SEQ ID NO:2 or a salt thereof, which includes (a) a cell containing a gene whose expression is controlled by said protein or a partial peptide thereof or a salt thereof, (b) said protein or a partial peptide thereof or a salt thereof, and (c) a polynucleotide capable of hybridizing to said gene under highly stringent conditions;
[11] the screening method described in [7], which comprises cultivating a cell having an ability to produce a protein comprising the same or substantially the same amino acid sequence as the amino acid sequence shown by SEQ ID NO:2 or a partial peptide thereof or a salt thereof in the presence and absence of a test substance, and comparing the activities of said protein or a partial peptide thereof or a salt thereof under the two conditions;
[12] a screening kit for a prophylactic or therapeutic substance for a disease associated with a protein comprising the same or substantially the same amino acid sequence as the amino acid sequence shown by SEQ ID NO:2 or a salt thereof, which includes (a) a cell having an ability to produce said protein or a partial peptide thereof or a salt thereof, and (b) a polynucleotide capable of hybridizing to a gene whose expression is controlled by said protein or a partial peptide thereof or a salt thereof under highly stringent conditions;
[13] a screening method for a prophylactic or therapeutic substance for a disease associated with a protein comprising the same or substantially the same amino acid sequence as the amino acid sequence shown by SEQ ID NO:2 or a salt thereof, which comprises using a polynucleotide comprising the base sequence that encodes said protein or a partial peptide thereof;
[14] a screening method for a prophylactic or therapeutic substance for a disease associated with a protein comprising the amino acid sequence shown by SEQ ID NO:2 or a salt thereof, which comprises using a polynucleotide comprising the base sequence that encodes said protein or a partial peptide thereof;
[15] the screening method described in [14] above, wherein the polynucleotide comprises the entire or a portion of the base sequence shown by SEQ ID NO:1;
[16] the screening method described in [14] above, wherein the disease is diabetes or a renal disease;
[17] the screening method described in [14] above, wherein the disease is diabetic nephropathy;
[18] the screening method described in [14] above, which comprises cultivating a cell having an ability to produce a protein comprising the same or substantially the same amino acid sequence as the amino acid sequence shown by SEQ ID NO:2 or a partial peptide thereof or a salt thereof in the presence and absence of a test substance, and comparing the amounts of mRNA that encodes said protein or a partial peptide thereof under the two conditions;
[19] a screening kit for a prophylactic or therapeutic substance for a disease associated with a protein comprising the same or substantially the same amino acid sequence as the amino acid sequence shown by SEQ ID NO:2 or a salt thereof, which includes (a) a cell having an ability to produce said protein or a partial peptide thereof or a salt thereof, and (b) a polynucleotide capable of hybridizing to mRNA that encodes said protein or a partial peptide thereof under highly stringent conditions;
[20] a prophylactic or therapeutic agent for a disease associated with a protein comprising the same or substantially the same amino acid sequence as the amino acid sequence shown by SEQ ID NO:2 or a salt thereof, which contains an antibody against said protein or a partial peptide thereof or a salt thereof;
[21] the prophylactic or therapeutic agent described in [20] above, wherein the disease is diabetes or a renal disease;
[22] the prophylactic or therapeutic agent described in [20] above, wherein the disease is diabetic nephropathy;
[23] a prophylactic or therapeutic agent for a disease associated with a protein comprising the same or substantially the same amino acid sequence as the amino acid sequence shown by SEQ ID NO:2 or a salt thereof, which contains a polynucleotide having a base sequence complementary to the base sequence that encodes said protein or a partial peptide thereof;
[24] the prophylactic or therapeutic agent described in [23] above, wherein the disease is diabetes or a renal disease;
[25] the prophylactic or therapeutic agent described in [23] above, wherein the disease is diabetic nephropathy;
[26] a diagnostic reagent for a disease associated with a protein comprising the same or substantially the same amino acid sequence as the amino acid sequence shown by SEQ ID NO:2 or a salt thereof, which contains an antibody against said protein or a partial peptide thereof or a salt thereof;
[27] the diagnostic reagent described in [26] above, wherein the disease is diabetes or a renal disease;
[28] the diagnostic reagent described in [26] above, wherein the disease is diabetic nephropathy;
[29] a diagnostic reagent for a disease associated with a protein comprising the same or substantially the same amino acid sequence as the amino acid sequence shown by SEQ ID NO:2 or a salt thereof, which contains a polynucleotide comprising the base sequence that encodes said protein or a partial peptide thereof;
[30] the diagnostic reagent described in [29] above, wherein the disease is diabetes or a renal disease;
[31] the diagnostic reagent described in [29] above, wherein the disease is diabetic nephropathy;
[32] a prophylactic or therapeutic agent for diabetes or a renal disease, which contains a TSC-22 suppressant;
[33] the prophylactic or therapeutic agent described in [32] above, wherein the renal disease is diabetic nephropathy;
[34] a prophylactic or therapeutic method for diabetes or a renal disease in a mammal, which comprises administering a TSC-22 suppressant to said mammal;
[35] the method described in [34] above, wherein the renal disease is diabetic nephropathy;
[36] use of a TSC-22 suppressant for the production of a prophylactic or therapeutic agent for diabetes or a renal disease;
[37] the use described in [36] above, wherein the renal disease is diabetic nephropathy, and the like.

### Brief Description of the Drawings

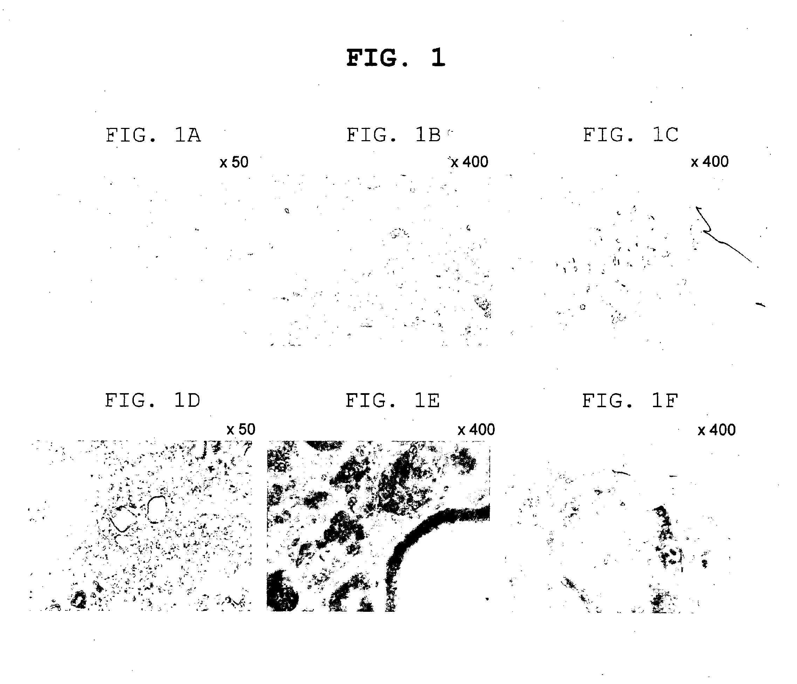

FIGS. 1A to F show how TSC-22 is expressed in the kidneys of spontaneously hypercholesterolemic SHC rats and normal SD rats. FIGS. 1A to C show sections from the kidneys of normal SD rats, and FIGS. 1D to F show sections from the kidneys of spontaneously hypercholesterolemic SHC rats. FIGS. 1A and D (magnification: x 50) and FIGS. 1B and E (magnification: x 400) are photomicrographs of the renal tubule, and FIGS. 1C and F (magnification: x 400) are photomicrographs of the glomerulus.

### Best Mode for Embodiment of the Invention

In the present invention, a protein comprising the same or substantially the same amino acid sequence as the amino acid sequence shown by SEQ ID NO:2 (hereinafter also abbreviated as "the protein of the present invention") may be a protein derived from a cell (for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like) of a mammal (for example, human, mouse, rat, rabbit, sheep, swine, bovine, horse, bird, cat, dog, monkey, chimpanzee, and the like), or any tissue where such cells are present, for example, brain, any portion of the brain (for example, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testicle, ovary, placenta, uterus, bone, joint, skeletal muscle, and the like, and may also be a chemically synthesized protein or a protein synthesized using a cell-free translation system. Alternatively, this protein may be a recombinant protein produced from a transformant introduced with a polynucleotide having the base sequence that encodes the above-described amino acid sequence.

As substantially the same amino acid sequence as the amino acid sequence shown by SEQ ID NO : 2, an amino acid sequence having a homology of about 70% or more, preferably about 80% or more, more preferably about 90% or more, particularly preferably about 95% or more, and most preferably about 98% or more, to the amino acid sequence shown by SEQ ID NO:2, and the like can be mentioned.

As preferable examples of the protein comprising substantially the same amino acid sequence as the amino acid sequence shown by SEQ ID NO:2, a protein that comprises substantially the same amino acid sequence as the amino acid sequence shown by SEQ ID NO:2 above, and that has substantially the same quality of activity as a protein comprising the amino acid sequence shown by SEQ ID NO:2, and the like, can be mentioned.

As examples of substantially the same quality of activity, activity to regulate the transcription of the insulin gene and the like, and the like can be mentioned. "Substantially the same quality" means that the proteins are qualitatively (e.g., physiologically or pharmacologically) equivalent to each other. Accordingly, it is preferable that the proteins be equivalent to each other in terms of transcription regulatory activity (e.g., about 0.01 to 100 times, preferably about 0.1 to 10 times, more preferably 0.5 to 2 times), but quantitative factors such as the extent of activity and protein molecular weight may be different.

A measurement of transcription regulatory activity can be conducted using a publicly known method, for example, Northern analysis of a target gene, gel shift assay and the like. Alternatively, the activity of the protein of the present invention can also be evaluated using a method based on the intracellular localization thereof, for example, examining the degree of migration from cytoplasm to nucleus.

Examples of the protein of the present invention also include what is called muteins of proteins comprising (i) an amino acid sequence having one or two or more amino acids (preferably about 1 to about 30, preferably about 1 to about 10, more preferably several (1 to 5) amino acids) deleted from the amino acid sequence shown by SEQ ID NO:2, (ii) an amino acid sequence having one or two or more amino acids (preferably about 1 to about 30, preferably about 1 to about 10, more preferably several (1 to 5) amino acids) added to the amino acid sequence shown by SEQ ID NO:2, (iii) an amino acid sequence having one or two or more amino acid (preferably about 1 to about 30, preferably about 1 to about 10, more preferably several (1 to 5) amino acids) inserted to the amino acid sequence shown by SEQ ID NO:2, (iv) an amino acid sequence having one or two or more amino acids (preferably about 1 to about 30, preferably about 1 to about 10, more preferably several (1 to 5) amino acids) substituted with other amino acids in the amino acid sequence shown by SEQ ID NO:2, or (v) an amino acid sequence comprising a combination thereof.

When an amino acid sequence is inserted, deleted or substituted as described above, the position of the insertion, deletion or substitution is not subject to limitation, as long as the protein retains its activity.

The protein of the present invention is preferably a protein having the amino acid sequence shown by SEQ ID NO:2, that is, the human TSC-22 protein or a homologue thereof in another mammal.

For the proteins mentioned herein, the left end is the N terminal (amino terminal) and the right end is the C terminal (carboxyl terminal) in accordance with the conventional peptide marking. Regarding the protein of the present invention, including a protein comprising the amino acid sequence shown by SEQ ID NO:2, the C terminal may be any of a carboxyl group (-COOH), a carboxylate (-COO⁻) , an amide (-CONH₂), and an ester (-COOR).

Here, as R in the ester, a C₁₋₆ alkyl group such as methyl, ethyl, n-propyl, isopropyl, and n-butyl; a C₃₋₈ cycloalkyl group such as cyclopentyl and cyclohexyl; a C₆₋₁₂ aryl group such as phenyl and α-naphthyl; a phenyl-C₁₋₂ alkyl group such as benzyl and phenethyl; a C₇₋₁₄ aralkyl group such as an α-naphthyl-C₁₋₂ alkyl group such as α-naphthylmethyl; a pivaloyloxymethyl group; and the like are used.

When the protein of the present has a carboxyl group (or a carboxylate) at a position other than the C terminal, a protein wherein the carboxyl group is amidated or esterified is also included in the protein of the present invention. In this case, as the ester, the above-described ester at the C terminal, and the like, for example, are used.

Furthermore, the protein of the present invention also includes a protein wherein the amino group of the N terminal amino acid residue (e.g., methionine residue) is protected by a protecting group (for example, C₁₋₆ acyl groups such as C₁₋₆ alkanoyl groups such as formyl group and acetyl group; and the like), a protein wherein the N terminal glutamine residue, which is produced upon cleavage in vivo, has been converted to pyroglutamic acid, a protein wherein a substituent (for example, -OH, -SH, amino group, imidazole group, indole group, guanidino group, and the like) on a side chain of an amino acid in the molecule is protected by an appropriate protecting group (for example, C₁₋₆ acyl groups such as C₁₋₆ alkanoyl groups such as formyl group and acetyl group; and the like), a conjugated protein such as what is called a glycoprotein having a sugar chain bound thereto, and the like.

A partial peptide of the protein of the present invention (hereinafter also simply abbreviated as "the partial peptide of the present invention") may be any peptide having the above-described partial amino acid sequence of the protein of the present invention, and having substantially the same quality of activity as the protein of the present invention; for example, a peptide having a partial amino acid sequence comprising the LZ domain and a conserved region adjoining to the amino terminal side thereof (TSC box), in the amino acid sequence shown by SEQ ID NO:2, and the like, are used.

Regarding the number of amino acids in the partial peptide of the present invention, a peptide having at least 30, preferably 60 or more, and more preferably 100 or more amino acids of the amino acid sequence that constitutes the protein of the present invention, and the like are preferred.

Here, "substantially the same quality of activity" has the same definition as above. A measurement of "substantially the same quality of activity" can be conducted in the same manner as above.

For the partial peptide of the present invention, the C terminal may be any of a carboxyl group (-COOH), a carboxylate (-COO⁻), an amide (-CONH₂), and an ester (-COOR). Here, as R in the ester, the same as those mentioned for the protein of the present invention above can be mentioned. When the partial peptide of the present invention has a carboxyl group (or a carboxylate) at a position other than the C terminal, a partial peptide wherein the carboxyl group is amidated or esterified is also included in the partial peptide of the present invention. In this case, as the ester, the above-described ester at the C terminal, and the like, for example, are used.

Furthermore, the partial peptide of the present invention also includes a protein wherein the amino group of the N terminal methionine residue is protected by a protecting group, a protein wherein Gln, which is produced upon cleavage at the N terminal in vivo, has been converted to pyroglutamic acid, a protein wherein a substituent on a side chain of an amino acid in the molecule is protected by an appropriate protecting group, a conjugated peptide such as what is called a glycopeptide having a sugar chain bound thereto, and the like, as with the above-described protein of the present invention.

As the salt of the protein of the present invention or a partial peptide thereof, physiologically acceptable salts with acid or base can be mentioned, and physiologically acceptable acid addition salts are preferred. Useful salts include, for example, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein of the present invention or a salt thereof can be produced from a cell or tissue of the aforementioned mammal by a method known per se of protein purification. Specifically, the protein of the present invention or a salt thereof can be produced by homogenizing mammalian tissue or cells, and separating and purifying the soluble fraction and/or nuclear fraction by a chromatography such as reversed-phase chromatography, ion exchange chromatography or affinity chromatography, and the like.

The protein of the present invention or a partial peptide thereof or a salt thereof (hereinafter also comprehensively referred to as "the protein or the like of the present invention") can also be produced according to a publicly known method of peptide synthesis.

The method of peptide synthesis may be any of, for example, a solid phase synthesis process and a liquid phase synthesis process. A desired protein can be produced by condensing a partial peptide or amino acid capable of constituting the protein of the present invention with the remaining portion, and removing any protecting group the resultant product may have.

Here, the condensation and the protecting group removal are conducted in accordance with methods known per se, for example, the methods indicated in (i) to (v) below:
(i) M. Bodanszky and M.A. Ondetti: Peptide Synthesis, Interscience Publishers, New York (1966)
(ii) Schroeder and Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975);
(iv) Haruaki Yajima and Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten.

The thus-obtained protein can be purified and isolated by a publicly known method of purification. Here, as examples of the method of purification, solvent extraction, distillation, column chromatography, liquid chromatography, recrystallization, a combination thereof, and the like can be mentioned.

When the protein obtained by the above-described method is a free form, it can be converted to an appropriate salt by a publicly known method or a method based thereon; conversely, when the protein is obtained in the form of a salt, the salt can be converted to a free form or another salt by a publicly known method or a method based thereon.

For the synthesis of the protein of the present invention or a partial peptide thereof or a salt thereof, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenylhydroxymethyl)phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired protein or peptide (hereinafter also generically referred to as "protein or the like") according to one of various methods of condensation known per se. At the end of the reaction, the protein or the like is cleaved from the resin and at the same time various protecting groups are removed, and a reaction to form an intramolecular disulfide bond is carried out in a highly diluted solution to obtain the desired protein or the like or an amide thereof.

For the above-described condensation of protected amino acids, various activation reagents which can be used for protein synthesis can be used, and a carbodiimide is preferably used. As the carbodiimide, DCC, N, N'-diisopropylcarbodiimide, N-ethyl-N'-(3-dimethylaminopropyl)carbodiimide and the like are used. For the activation using these carbodiimides, the protected amino acid, along with a racemation-suppressing additive (for example, HOBt, HOOBt), may be added directly to the resin, or the protected amino acid may be activated in advance as a symmetric acid anhydride or HOBt ester or HOOBt ester and then added to the resin.

Solvents used for the activation of protected amino acids and condensation thereof with a resin can be appropriately selected from among solvents that are known to be usable for protein condensation reactions. As examples of useful solvents, acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; amines such as pyridine; ethers such as dioxane and tetrahydrofuran; nitriles such as acetonitrile and propionitrile; esters such as methyl acetate and ethyl acetate; suitable mixtures thereof; and the like can be mentioned. Reaction temperature is appropriately selected from the range that is known to be usable for protein binding reactions, and is normally selected from the range of about -20°C to about 50°C. An activated amino acid derivative is normally used from 1.5 to 4 times in excess. When a test using the ninhydrin reaction reveals that the condensation is insufficient, sufficient condensation can be conducted by repeating the condensation reaction without elimination of protecting groups. If the condensation is insufficient even though the reaction is repeated, unreacted amino acids may be acetylated using acetic anhydride or acetylimidazole.

A protecting method and a protecting group for a functional group that should not be involved in the reaction of raw materials, a method of removing the protecting group, a method of activating a functional group involved in the reaction, and the like can be appropriately selected from among publicly known groups or publicly known means.

As examples of the protecting group for an amino group of the starting material, Z, Boc, tertiary pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc, and the like can be used.

A carboxyl group can be protected, for example, by alkyl esterification (for example, linear, branched or cyclic alkyl esterification with methyl, ethyl, propyl, butyl, tertiary butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation, and the like.

The hydroxyl group of serine can be protected by, for example, esterification or etherification. As examples of a group suitable for this esterification, lower alkanoyl groups such as an acetyl group, aroyl groups such as a benzoyl group, and groups derived from carbonic acid such as a benzyloxycarbonyl group and an ethoxycarbonyl group, and the like are used. As examples of a group suitable for etherification, a benzyl group, a tetrahydropyranyl group, a t-butyl group, and the like can be mentioned.

As examples of the protecting group for the phenolic hydroxyl group of tyrosine, Bzl, Cl₂-Bzl, 2-nitrobenzyl, Br-Z, tertiary butyl, and the like can be used.

As examples of the protecting group for the imidazole of histidine, Tos, 4-methoxy-2,3,6-trimethylbenzenesulfonyl, DNP, benzyloxymethyl, Bum, Boc, Trt, Fmoc, and the like are used.

As examples of the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like are used. The elimination reaction by the above-described acid treatment is generally carried out at a temperature of about -20°C to about 40°C; the acid treatment is efficiently conducted by adding a cation scavenger, for example, anisole, phenol, thioanisole, m-cresol, p-cresol, dimethylsulfide, 1,4-butanedithiol and 1,2-ethanedithiol. Also, a 2,4-dinitrophenyl group used as a protecting group of the imidazole of histidine is removed by thiophenol treatment; a formyl group used as a protecting group of the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As examples of those obtained by activation of the carboxyl group in the starting material, a corresponding acid anhydride, an azide, an activated ester [an ester with an alcohol (for example, pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like are used. As examples of those obtained by activation of the amino group in the starting material, a corresponding phosphoric amide is used.

In another method of preparing an amide of a protein or the like, for example, the α-carboxyl group of the carboxy terminal amino acid is first amidated and hence protected, and a peptide (protein) chain is elongated to a desired chain length toward the amino group side, thereafter a protein or the like having the protecting group for the N terminal α-amino group of the peptide chain only removed and a protein or the like having the protecting group for the C terminal carboxyl group only removed are prepared, and these proteins or the like are condensed in a mixed solvent described above. For details about the condensation reaction, the same as above applies. After the protected protein or the like obtained by the condensation is purified, all protecting groups can be removed by the above-described method to yield a desired crude protein or the like. By purifying this crude protein or the like using various publicly known means of purification, and freeze-drying the main fraction, a desired amide of the protein or the like can be prepared.

In order to obtain esters of the protein or the like, a desired ester of the protein or the like can be prepared by, for example, condensing the α-carboxyl group of the carboxy terminal amino acid with a desired alcohol to yield an amino acid ester, and then treating the ester in the same manner as with an amide of the protein or the like.

The partial peptide of the present invention or a salt thereof can also be produced by cleaving the protein of the present invention or a salt thereof with an appropriate peptidase.

Furthermore, the protein of the present invention or a partial peptide thereof or a salt thereof can also be produced by cultivating a transformant comprising DNA that encodes the protein of the present invention or a partial peptide thereof, and separating and purifying the protein or the like of the present invention from the culture obtained.

As the DNA that encodes the protein of the present invention or a partial peptide thereof, genomic DNA, a genomic DNA library, cDNA derived from any cell (for example, splenocyte, nerve cell, glial cell, pancreatic β cell, myelocyte, mesangial cell, Langerhans' cell, epidermal cell, epithelial cell, endothelial cell, fibroblast, fibrocyte, myocyte, adipocyte, immune cell (for example, macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like) of a human or another mammal (for example, bovine, monkey, horse, swine, sheep, goat, dog, cat, guinea pig, rat, mouse, rabbit, hamster, and the like), a blood cell system cell, or any tissue where such cells are present, for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, peripheral blood cells, prostate, testicle, testis, ovary, placenta, uterus, bone, joint, skeletal muscle, and the like (particularly the brain or any portion of the brain), a cDNA library derived from the aforementioned cell or tissue, synthetic DNA and the like can be mentioned. The vector used for the library may be any of a bacteriophage, a plasmid, a cosmid, a phagemid and the like. The DNA can also be amplified directly by a reverse transcriptase polymerase chain reaction (hereinafter abbreviated as "RT-PCR method") using a total RNA or mRNA fraction prepared from the above-described cell or tissue.

As examples of the DNA that encodes the protein of the present invention, DNA comprising the base sequence shown by SEQ ID NO:1, DNA that comprises a base sequence hybridizing to the base sequence shown by SEQ ID NO:1 under highly stringent conditions, and that encodes the aforementioned protein having substantially the same quality of activity (e.g., transcription regulatory activity and the like) as a protein comprising the amino acid sequence shown by SEQ ID NO:2, and the like can be mentioned.

As examples of the DNA capable of hybridizing to the base sequence shown by SEQ ID NO:1 under highly stringent conditions, DNA that comprises a base sequence showing a homology of about 50% or more, preferably about 60% or more, more preferably about 70% or more, particularly preferably about 80% or more, and most preferably about 90% or more, to the base sequence shown by SEQ ID NO:1, and the like are used.

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions.

High-stringent conditions refer to, for example, conditions involving a sodium concentration of about 19 to 40mM, preferably about 19 to 20mM, and a temperature of about 50 to 70°C, preferably about 60 to 65°C. In particular, a case wherein the sodium concentration is about 19mM and the temperature is about 65°C is preferred.

The DNA that encodes the protein of the present invention is preferably DNA comprising the base sequence shown by SEQ ID NO:1 and the like.

The DNA that encodes the partial peptide of the present invention may be any one comprising the base sequence that encodes the same or substantially the same amino acid sequence as a portion of the amino acid sequence shown by SEQ ID NO:2. The DNA may be any of genomic DNA, a genomic DNA library, cDNA derived from the above-described cell or tissue, a cDNA library derived from the above-described cell or tissue, and synthetic DNA. The vector used for the library may be any of a bacteriophage, a plasmid, a cosmid, a phagemid and the like. The DNA can also be amplified directly by the RT-PCR method using an mRNA fraction prepared from the above-described cell or tissue.

Specifically, as examples of the DNA that encodes the partial peptide of the present invention, (1) DNA that comprises a partial base sequence of DNA comprising the base sequence shown by SEQ ID NO:1, (2) DNA that comprises a base sequence hybridizing to DNA comprising the base sequence shown by SEQ ID NO:1 under highly stringent conditions, and that encodes a peptide having substantially the same quality of activity (e.g., transcription regulatory activity and the like) as that of a protein comprising the amino acid sequence encoded by the DNA and the like are used.

As examples of the DNA capable of hybridizing to the base sequence shown by SEQ ID NO:1 under highly stringent conditions, a polynucleotide comprising a base sequence showing a homology of about 60% or more, preferably about 70% or more, more preferably about 80% or more, and most preferably about 90% or more, to the base sequence, and the like are used.

The DNA that encodes the protein of the present invention or a partial peptide thereof can be cloned by amplifying it by the PCR method using a synthetic DNA primer comprising a portion of the base sequence that encodes the protein or peptide, or by hybridizing DNA incorporated in an appropriate expression vector to a labeled DNA fragment or synthetic DNA that encodes a portion or the entire region of the protein of the present invention. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known per se, such as the ODA-LA PCR method, the Gapped duplex method, the Kunkel method and the like, or a method based thereon, using a publicly known kit, for example, Mutant™-super Express Km (Takara Shuzo Co., Ltd.), Mutan™-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

A DNA expression vector that encodes the protein of the present invention can be produced by, for example, cutting out a desired DNA fragment from the DNA that encodes the protein of the present invention, and joining the DNA fragment downstream of a promoter in an appropriate expression vector.

Useful expression vectors include plasmids derived from *Escherichia* coli (e.g., pBR322, pBR325, pUC12, pUC13); plasmids derived from *Bacillus subtilis* (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo, and the like.

The promoter may be any promoter, as long as it is appropriate for the host used to express the gene.

For example, when the host is an animal cell, the SRα promoter, the SV40 promoter, the LTR promoter, the CMV (cytomegalovirus) promoter, the HSV-TK promoter and the like are used. Of these, the CMV promoter, the SRα promoter and the like are preferred.

When the host is a bacterium of the genus *Escherichia,* the trp promoter, the lac promoter, the recA promoter, the λP_{L} promoter, the lpp promoter, the T7 promoter and the like are preferred.

When the host is a bacterium of the genus *Bacillus,* the SPO1 promoter, the SPO2 promoter, the penP promoter and the like are preferred.

When the host is yeast, the PHO5 promoter, the PGK promoter, the GAP promoter, the ADH promoter and the like are preferred.

When the host is an insect cell, the polyhedrin prompter, the P10 promoter and the like are preferred.

Useful expression vectors include, in addition to the above, those optionally harboring an enhancer, a splicing signal, a polyA addition signal, a selection marker, an SV40 replication origin (hereinafter also abbreviated as SV40ori) and the like. As examples of the selection marker, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance) and the like can be mentioned. In particular, when a Chinese hamster cell lacking the dhfr gene is used in combination with the dhfr gene as the selection marker, a target gene can also be selected using a thymidine-free medium.

In addition, as required, a signal sequence that matches the host may be added to the N terminal side of the protein of the present invention. Useful signal sequences include a PhoA signal sequence, an OmpA signal sequence and the like when the host is a bacterium of the genus *Escherichia;* an α-amylase signal sequence, a subtilisin signal sequence and the like when the host is a bacterium of the genus *Bacillus;* an MFα signal sequence, an SUC2 signal sequence and the like when the host is yeast; and an insulin signal sequence, an α-interferon signal sequence, an antibody molecule signal sequence and the like when the host is an animal cell.

A transformant comprising the thus-obtained "DNA that encodes the protein of the present invention" can be produced by transforming the host with an expression vector comprising the DNA according to a publicly known method.

Here, as the expression vector, those mentioned above can be mentioned.

Useful hosts include, for example, a bacterium of the genus *Escherichia,* a bacterium of the genus *Bacillus,* yeast, an insect cell, an insect, an animal cell and the like.

Useful bacteria of the genus *Escherichia* include, for example, *Escherichia coli* K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)) and the like.

Useful bacteria of the genus *Bacillus* include, for example, *Bacillus subtilis* MI114 (Gene, Vol. 24, 255 (1983)), 207-21 (Journal of Biochemistry, Vol. 95, 87 (1984)) and the like.

Useful yeasts include, for example, *Saccharomyces cerevisiae* AH22, AH22R⁻, NA87-11A, DKD-5D and 20B-12, *Schizosaccharomyces pombe* NCYC1913 and NCYC2036, *Pichia pastoris* KM71, and the like.

Useful insect cells include, for example, *Spodoptera frugiperda* cell (Sf cell), MG1 cell derived from the mid-intestine of *Trichoplusia* ni, High Five™ cell derived from an egg of *Trichoplusia ni,* cell derived from Mamestra brassicae, cell derived from Estigmena acrea, and the like can be mentioned when the virus is AcNPV. When the virus is BmNPV, useful insect cells include *Bombyx mori* N cell (BmN cell) and the like. Useful Sf cells include, for example, Sf9 cell (ATCC CRL1711), Sf21 cell (both in Vaughn, J. L. et al., In Vivo, 13, 213-217 (1977) and the like.

Useful insects include, for example, a larva of *Bombyx mori* (Maeda et al., Nature, Vol. 315, 592 (1985)) and the like.

Useful animal cells include, for example, monkey cell COS-7, Vero, Chinese hamster cell CHO (hereafter abbreviated as CHO cell), Chinese hamster cell lacking the dhfr gene CHO (hereafter abbreviated as CHO(dhfr⁻) cell), mouse L cell, mouse AtT-20, mouse myeloma cell, rat GH3, human FL cell and the like.

Transformation can be carried out according to the kind of host in accordance with a publicly known method.

A bacterium of the genus *Escherichia* can be transformed, for example, in accordance with a method described in Proc. Natl. Acad. Sci. U.S.A., Vol. 69, 2110 (1972), Gene, Vol. 17, 107 (1982) and the like.

A bacterium of the genus *Bacillus* can be transformed, for example, according to a method described in Molecular and General Genetics, Vol. 168, 111 (1979) and the like.

Yeast can be transformed, for example, in accordance with a method described in Methods in Enzymology, Vol. 194, 182-187 (1991), Proc. Natl. Acad. Sci. USA, Vol. 75, 1929 (1978) and the like.

An insect cell and an insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably contains a carbon source, a nitrogen source, an inorganic substance and the like necessary for the growth of the transformant. Here, as examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, potato extract and the like can be mentioned; as examples of the inorganic substance, calcium chloride, sodium dihydrogen phosphate, magnesium chloride and the like can be mentioned. In addition, the medium may be supplemented with yeast extract, vitamins, growth promoting factor and the like. Preferably, the pH of the medium is about 5 to about 8.

As a example of the medium used to cultivate a transformant whose host is a bacterium of the genus *Escherichia,* a M9 medium supplemented with glucose and a casamino acid (Miller, Journal of Experiments in Molecular Genetics, 431-433, Cold Spring Harbor Laboratory, New York, 1972) can be mentioned. As required, in order to increase promoter efficiency, a chemical agent such as 3β-indolylacrylic acid may be added to the medium.

Cultivation of a transformant whose host is a bacterium of the genus *Escherichia* is normally carried out at about 15°C to about 43°C for about 3 to about 24 hours. As necessary, the culture may be aerated or agitated.

Cultivation of a transformant whose host is a bacterium of the genus *Bacillus* is normally carried out at about 30°C to about 40°C for about 6 to about 24 hours. As necessary, the culture may be aerated or agitated.

As examples of the medium for cultivating a transformant whose host is a yeast, Burkholder's minimum medium [Bostian, K.L. et al., Proc. Natl. Acad. Sci. USA, vol. 77, 4505 (1980)] and SD medium supplemented with 0.5% casamino acid [Bitter, G.A. et al., Proc. Natl. Acad. Sci. USA, vol. 81, 5330 (1984)] can be mentioned. The medium's pH is preferably about 5 to 8. Cultivation is normally carried out at about 20°C to about 35°C for about 24 to about 72 hours. As necessary, the culture may be aerated or agitated.

Useful medium for cultivating a transformant whose host is an insect cell or an insect include, for example, Grace's insect medium [Grace, T.C.C., Nature, 195, 788 (1962)] supplemented with additives such as inactivated 10% bovine serum as appropriate. The medium's pH is preferably about 6.2 to 6.4. Cultivation is normally carried out at about 27°C for about 3 to 5 days. As necessary, the culture may be aerated or agitated.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally carried out at about 30°C to 40°C for about 15 to 60 hours. As necessary, the culture may be aerated or agitated.

As described above, the protein of the present invention can be produced in a cell (in the nucleus or in cytoplasm) of the transformant or outside the cell.

The protein or the like of the present invention can be separated and purified from the culture obtained by cultivating the aforementioned transformant according to a method known per se.

For example, when the protein or the like of the present invention is extracted from a cultured bacterum or a cell cytoplasm, a method is used as appropriate wherein bacteria or cells are collected by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100™. On the other hand, when the protein or the like of the present invention is extracted from a nuclear fraction, a method of preparing a crude extract of the nuclear protein by treating the precipitate from the above-described centrifugation or filtration with, for example, a hypertonic solution and the like, and recovering the supernatant via centrifugation, and the like are used.

Isolation and purification of the protein or the like of the present invention contained in the thus-obtained soluble fraction or nuclear extract can be conducted according to a method know per se. Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained protein or the like is a free form, it can be converted to a salt by a method known per se or a method based thereon; when the protein or the like is obtained as a salt, it can be converted to a free form or another salt by a method known per se or a method based thereon.

Note that the protein or the like produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained protein or the like of the present invention can be confirmed by enzyme immunoassay, Western blotting and the like using a specific antibody.

Furthermore, the protein or the like of the present invention can also be synthesized by in vitro translation using a cell-free protein translation system comprising a rabbit reticulocyte lysate, wheat germ lysate, *Escherichia coli* lysate and the like, with RNA corresponding to the above-described DNA that encodes the protein of the present invention or a partial peptide thereof as the template. Alternatively, the protein or the like of the present invention can be synthesized using a cell-free transcription/translation system containing RNA polymerase, with the DNA that encodes the protein of the present invention or a partial peptide thereof as the template.

As the disease associated with the protein of the present invention or a salt thereof, a disease characterized by an increased or decreased amount of the protein of the present invention or a salt thereof compared to normal cases can be mentioned.

Here, as examples of the "disease characterized by an increased amount of the protein of the present invention or a salt thereof compared to normal cases", renal diseases (for example, diabetic nephropathy; chronic glomerulonephritis; IgA nephropathy; chronic graft rejection after renal transplantation; renal cancer; peritoneal sclerosis during peritoneal dialysis; acute nephritic syndrome; nephrotic syndrome; focal glomerulosclerosis; membranous nephropathy; diabetes insipidus; chronic pyelonephritis; progressive renal disease); endocrine/metabolic diseases (for example, diabetes); cardiovascular diseases (for example, arteriosclerosis; myocardial infarction; heart failure; cardiomyopathy; vascular restenosis after PTCA and stenting; chronic graft rejection after heart/vessel transplantation; thrombosis); cerebrovascular disorders (for example, cerebral infarction); pulmonary diseases (for example, pulmonary fibrosis, chronic obstructive plumonary disease, lung cancer); hepatic diseases (for example, liver cirrhosis, hepatitis, liver cancer); gastrointestinal diseases (for example, colitis, colon cancer); gonadal diseases (for example, prostatic cancer); collagen diseases (for example, scleroderma, systemic lupus erythematosus); rheumatic diseases (for example, rheumatoid arthritis); bone diseases (for example, osteoporosis) and the like can be mentioned.

As examples of the "disease characterized by a decreased amount of the protein of the present invention or a salt thereof compared to normal cases", gastrointestinal diseases (for example, gastric ulcer, duodenal ulcer; gastric cancer; salivary gland cancer); dermal diseases (for example, burns, postoperative wounds); brain tumors and the like can be mentioned.

The disease associated with the protein of the present invention or a salt thereof is preferably a renal disease or diabetes, more preferably diabetic nephropathy.

The present invention relates to a screening method for a prophylactic or therapeutic substance for a disease associated with the protein of the present invention, which comprises using said protein or the like. This screening method is conducted by, for example,
1) cultivating a cell having an ability to produce the protein or the like of the present invention in the presence and absence of a test substance, and comparing the amounts of the protein or the like of the present invention produced under the two conditions;
2) comparing the activities of the protein or the like of the present invention in the presence and absence of a test substance; and the like.

The latter method can be further classified into:
2a) a method of evaluating the activity of the protein or the like of the present invention by measuring the expression of a gene whose expression is controlled by the protein or the like in a cell containing the gene;
2b) a method of evaluating the activity of the protein or the like of the present invention by measuring the degree of binding of the protein or the like or a transcription regulatory factor capable of interacting therewith to the cis-element of a promoter whose transcription activity is controlled by the protein or the like;
2c) a method of evaluating the activity of the protein or the like of the present invention by measuring the intracellular localization of the protein or the like, and the like.

As examples of the "gene whose expression is controlled by the protein or the like of the present invention", the insulin gene and the like can be mentioned. Alternatively, the gene may be chimeric DNA containing the cis-element of a promoter whose transcription activity is controlled by the protein or the like (e.g., insulin gene promoter and the like), and in this case, DNA that encodes various reporter proteins may be joined downstream of the promoter. As examples of the "cell containing a gene whose expression is controlled by the protein or the like of the present invention", pancreatic cells and the like can be mentioned. As examples of the "promoter whose transcription activity is controlled by the protein or the like of the present invention", the insulin gene promoter and the like can be mentioned. As examples of the "the transcription regulatory factor capable of interacting with the protein or the like of the present invention", a transcription regulatory factor capable of binding to the cis-element of the insulin gene promoter and the like can be mentioned, and the transcription regulatory factor can be provided as, for example, pancreatic cell nuclear extract. Alternatively, the transcription regulatory factor capable of interacting with the protein or the like of the present invention can be purified from the nuclear extract with the protein or the like of the present invention as the probe, or cDNA can be cloned by the yeast two-hybrid method using a polynucleotide that encodes the protein or the like of the present invention.

In the case of 2a) above, supply of the protein or the like of the present invention to the screening system can also be conducted using a cell further having an ability to produce the protein or the like.

Although "the cell having an ability to produce the protein or the like of the present invention" used in the screening method using the protein or the like of the present invention is not subject to limitation, it is preferably one wherein production of the protein or the like of the present invention is induced in response to various stimuli such as oxidative stress and growth factor treatment. Alternatively, the cell having an ability to produce the protein or the like of the present invention may be a transformant containing the aforementioned DNA that encodes the protein or the like of the present invention.

As preferable examples of the cell having an ability to produce the protein or the like of the present invention, cells isolated from the kidney or pancreas, preferably from the kidney, of a mammal (preferably human, rat, mouse and the like) can be mentioned. These cells may be immortalized cells.

Cultivation of the cell having an ability to produce the protein or the like of the present invention is conducted in the same manner as with the aforementioned transformant.

As examples of the test substance, a peptide, a protein, a non-peptide compound, a synthetic compound, a fermentation product, a cell extract, a plant extract, an animal tissue extract and the like can be mentioned.

The protein or the like of the present invention can be quantified using a publicly known method, for example, an antibody against the protein or the like of the present invention, according to a method such as Western analysis or ELISA or a method based thereon.

The "antibody against the protein or the like of the present invention" used here may be any of a monoclonal antibody and a polyclonal antibody, as long as it is capable of recognizing the protein or the like of the present invention. In addition, the antibody may be the antibody molecule as is, or the F(ab')₂, Fab' or Fab fraction of the antibody molecule. Furthermore, the antibody may have been labeled.

As examples of the labeling agent used for antibody labeling, a radioisotope, an enzyme, a fluorescent substance, a luminescent substance and the like can be used. As examples of the radioisotope, [³⁵S], [¹²⁵I] , [¹³¹I], [³H], [¹⁴C] and the like can be used. As the enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-(strepto)avidin system can also be used for binding of an antibody or an antigen and a labeling agent.

Alternatively, the protein or the like of the present invention can be quantified according to West-Western analysis or a method based thereon using the above-described transcription regulatory factor capable of interacting with the protein or the like of the present invention. The transcription regulatory factor has preferably been labeled, and as the labeling agent, the same substances as those described above can be used.

Alternatively, the protein or the like of the present invention can be quantified according to the gel shift assay method or a method based thereon using a polynucleotide to which it can bind. As the "polynucleotide to which the protein or the like of the present invention can bind", a polynucleotide comprising a sequence in the promoter region of a gene whose transcription is controlled by the protein of the present invention bound thereto can be mentioned. Previously reported polynucleotides include, for example, but are not limited to, the GC element of the type C natriuretic peptide gene.

In quantifying the protein or the like of the present invention, the quantified protein or the like may be a protein contained in a cell, a secreted protein outside a cell, or the sum of both.

When the protein or the like of the present invention contained in a cell is quantified, this quantitation is preferably conducted after the cell is treated with an appropriate fixative or membrane permeation promoting agent. It is also possible to quantify the protein or the like in a disrupted liquid obtained by suspending the cell in an appropriate buffer solution, and disrupting the cell by ultrasonication or freeze-drying and the like. Quantitation of the protein or the like may be conducted after the protein or the like in the disrupted liquid is separated and purified as necessary. When the protein or the like of the present invention that has migrated to the nucleus is quantified, the cell is disrupted in the same manner as above, the precipitate is recovered and treated with a hypertonic solution and the like, and the protein or the like in the thus-obtained nuclear extract is measured.

In the case of 2c) above, the intracellular localization of the protein or the like of the present invention can be evaluated by, for example, monitoring the degree of migration of the protein or the like from cytoplasm to nucleus in the cell having an ability to produce the protein or the like. For example, by immunostaining the cell with a fluorescently labeled antibody against the protein or the like of the present invention, the migration of the protein or the like from cytoplasm to nucleus can be monitored. Alternatively, it is also possible to directly monitor the migration of the protein or the like of the present invention from cytoplasm to nucleus using a transformant capable of expressing the protein or the like of the present invention in the form of a fusion protein with a fluorescent protein such as GFP (see, for example, Biochem. Biophys. Res. Commun., 278: 659-664 (2000)).

The present invention also relates to a screening kit that can be used for the screening method 1) above, which employs the amount produced of the protein or the like of the present invention as an index. The kit includes as its constituents at least one of, preferably both of (a) the above-described cell having an ability to produce the protein or the like of the present invention, and (b) a reagent capable of detecting the protein or the like of the present invention, for example, the above-described antibody against the protein or the like of the present invention, a polynucleotide to which the protein or the like can bind, or a transcription regulatory factor capable of interacting with the protein or the like. The kit may further include as required other reagents and instruments necessary or preferable for the conduct of the screening method 1) above.

As examples of the activity of the protein or the like of the present invention, used as an index in the screening method 2) above using the protein or the like of the present invention, transcription regulatory activity and the like can be mentioned. Specifically, for example, "activity to control the binding of a transcription regulatory factor capable of interacting with the protein or the like of the present invention to a target polynucleotide", "activity to bind the protein or the like of the present invention to a target polynucleotide", "activity to control the expression of a gene under the control of transcription by the protein or the like of the present invention", "nuclear migration activity of the protein or the like of the present invention" and the like can be mentioned.

As examples of "the transcription regulatory factor capable of interacting with the protein or the like of the present invention", a protein that is a transcription factor involved in the transcription control of the insulin gene, wherein the transcription regulatory activity is controlled by the protein of the present invention, and the like can be mentioned. Such a transcription regulatory factor can be provided as a nuclear extract of a cell that expresses it (for example, pancreatic cell and the like, in the case of the above-described insulin gene transcription factor).

As examples of "the target polynucleotide for the transcription regulatory factor capable of interacting with the protein or the like of the present invention", a polynucleotide comprising the base sequence of the insulin gene promoter derived from a human or another mammal, or a portion thereof, and the like can be mentioned.

As "the target polynucleotide of the protein or the like of the present invention", those mentioned to exemplify the above-described "polynucleotide to which the protein or the like of the present invention can bind" can be mentioned.

The above-described binding activity or binding-controlling activity can be measured using a publicly known method such as the gel shift assay method (electrophoretic mobility shift assay) or a method based thereon.

As "the gene under the control of transcription control by the protein or the like of the present invention", a gene under the control of a promoter containing as the cis-element a target polynucleotide of a transcription regulatory factor capable of interacting with the protein or the like of the present invention, or a gene under the control of a promoter containing as the cis-element a target polynucleotide of the protein or the like of the present invention, can be mentioned. As examples of the former, the insulin gene and the like can be mentioned. Previously reported examples of the latter gene include the type C natriuretic peptide gene.

The expression regulatory activity of such a gene can be measured by preparing an appropriate primer on the basis of the base sequence of the mRNA from the gene, and measuring the amount of the transcription product of the gene by RT-PCR. The above-described expression regulatory activity can also be measured according to a publicly known method or a method based thereon, for example, a Northern blotting method using a probe prepared by labeling a polynucleotide that comprises the entire or a portion of the base sequence of the mRNA produced from the gene. The above-described expression regulatory activity can also be measured according to a publicly known method or a method based thereon, by preparing an expression vector wherein an appropriate reporter gene joined downstream of a promoter containing a target polynucleotide of a transcription regulatory factor capable of interacting with the protein or the like of the present invention, or a target polynucleotide of the protein or the like of the present invention, introducing the vector to an appropriate cell, preferably a cell that produces the protein or the like of the present invention and/or a transcription regulatory factor capable of interacting therewith, and confirming the expression of the protein encoded by the reporter gene.

The present invention also relates to a screening kit that can be used for the screening method 2) above using the activity of the protein or the like of the present invention as an index. As examples of the kit that can be used for the screening method 2a) above, one including as its constituents at least one of, preferably all of (a) a cell containing a gene whose expression is controlled by the protein or the like of the present invention, (b) the protein or the like of the present invention and (c) a reagent capable of detecting the expression of the gene, for example a polynucleotide capable of hybridizing to the gene under highly stringent conditions, can be mentioned. The protein or the like of the present invention of (b) may be one produced by the cell (a).

As the kit that can be used for the screening method 2b) above, one including as its constituents at least one of, preferably both of (a) the protein or the like of the present invention and (b) the above-described polynucleotide to which the protein or the like of the present invention can bind, or a transcription regulatory factor capable of interacting with the protein or the like of the present invention, can be mentioned.

As the kit that can be used for the screening method 2c) above, one including as its constituents (a) a cell having an ability to produce the protein or the like of the present invention and, as required, (b) an antibody against the protein or the like, can be mentioned. Provided that the protein or the like of the present invention is produced in a form that can be visualized in a viable cell, like a fusion protein with a fluorescent protein such as GFP, in the cell (a), the antibody (b) may be omitted.

As required, these kits may further include as their constituents other reagents and instruments necessary or preferable for the conduct of the screening method 2) above.

In the above-described screening method, it is possible to diagnose whether a subject animal suffers from a disease associated with the protein of the present invention, or whether the animal is likely to suffer from the disease in the future, by comparing the amount or activity of the protein of the present invention in a cell or another sample from an animal suspected of suffering from a disease associated with the protein of the present invention or a salt thereof and that in a cell or another sample from a normal control animal, rather than by comparing the amounts produced or activities of the protein of the present invention in the presence and absence of a test substance.

By the screening method using the protein or the like of the present invention, a prophylactic or therapeutic substance for a disease associated with the protein of the present invention or a salt thereof, i.e., a substance that regulates (promotes or inhibits) the production of the protein or the like of the present invention, or a substance that regulates (promotes or inhibits) the activity of the protein or the like of the present invention can be screened for.

For example, a test substance that increases the amount produced of the protein or the like of the present invention by about 20% or more, preferably 30% or more, more preferably about 50% or more, as a substance for promoting the production of the protein or the like of the present invention; and a test substance that decreases the amount produced of the protein or the like of the present invention by about 20% or more, preferably 30% or more, more preferably about 50% or more, as a substance for inhibiting the production of the protein or the like of the present invention, can be selected respectively.

Also, for example, a test substance that increases the activity of the protein or the like of the present invention by about 20% or more, preferably 30% or more, more preferably about 50% or more, as a substance for promoting the activity of the protein or the like of the present invention; and a test substance that decreases the activity of the protein or the like of the present invention by about 20% or more, preferably 30% or more, more preferably about 50% or more, as a substance for inhibiting the activity of the protein or the like of the present invention, can be selected respectively.

The prophylactic or therapeutic substance for a disease associated with the protein of the present invention, obtained using the screening method of the present invention, may be any of a peptide, a protein, a non-peptide compound, a synthetic compound, a fermentation product, a cell extract, a plant extract, an animal tissue extract, plasma and the like. These may have formed a salt, and as specific examples of the salt, the same as the aforementioned salts of the protein of the present invention can be mentioned.

"A prophylactic or therapeutic substance for a disease associated with the protein of the present invention or a salt thereof" (compound), obtained by the screening method using the protein or the like of the present invention, can be used as a prophylactic or therapeutic agent for a disease associated with the protein of the present invention or a salt thereof after it is mixed with a pharmacologically acceptable carrier as required to yield a pharmaceutical composition.

Here, as examples of the pharmacologically acceptable carrier, various organic or inorganic carrier substances conventionally used as pharmaceutical preparation materials can be mentioned, and these are formulated as excipients, lubricants, binders and disintegrants, in solid preparations; as solvents, solubilizing agents, suspending agents, isotonizing agents, buffering agents and soothing agents, in liquid preparations, and the like. Also, as necessary, pharmaceutical preparation additives such as antiseptics, antioxidants, coloring agents, sweeteners and the like can be used.

As examples of suitable excipients, lactose, saccharose, D-mannitol, D-sorbitol, starch, gelatinized starch, dextrin, crystalline cellulose, low substituted hydroxypropyl cellulose, sodium carboxymethyl cellulose, gum arabic, pullulan, light silicic anhydride, synthetic aluminum silicate, magnesium metasilicate aluminate and the like can be mentioned.

As examples of suitable lubricants, magnesium stearate, calcium stearate, talc, colloidal silica and the like can be mentioned.

As examples of suitable binders, gelatinized starch, sucrose, gelatin, gum arabic, methyl cellulose, carboxymethyl cellulose, sodium carboxymethyl cellulose, crystalline cellulose, saccharose, D-mannitol, trehalose, dextrin, pullulan, hydroxypropyl cellulose, hydroxypropylmethyl cellulose, polyvinyl pyrrolidone and the like can be mentioned.

As examples of suitable disintegrants, lactose, saccharose, starch, carboxymethyl cellulose, calcium carboxymethyl cellulose, sodium crosscarmellose, sodium carboxymethyl starch, light silicic anhydride, low substituted hydroxypropyl cellulose and the like can be mentioned.

As examples of suitable solvents, water for injection, physiological saline, Ringer's solutions, alcohols, propylene glycol, polyethylene glycol, sesame oil, corn oil, olive oil, cottonseed oil and the like can be mentioned.

As examples of suitable solubilizing agents, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of suitable suspending agents, surfactants such as stearyl triethanolamine, sodium lauryl sulfate, lauryl aminopropionic acid, lecithin, benzalkonium chloride, benzethonium chloride and glyceryl monostearate; hydrophilic polymers such as polyvinyl alcohol, polyvinyl pyrrolidone, sodium carboxymethyl cellulose, methyl cellulose, hydroxymethyl cellulose, hydroxyethyl cellulose and hydroxypropyl cellulose; polysorbates, polyoxyethylene hardened castor oil and the like can be mentioned.

As examples of suitable isotonizing agents, sodium chloride, glycerin, D-mannitol, D-sorbitol, glucose and the like can be mentioned.

As examples of suitable buffers, buffer solutions of a phosphate, an acetate, a carbonate, a citrate and the like, and the like can be mentioned.

As examples of suitable soothing agents, benzyl alcohol and the like can be mentioned.

As examples of suitable antiseptics, paraoxybenzoates, chlorobutanol, benzyl alcohol, phenethyl alcohol, dehydroacetic acid, sorbic acid and the like can be mentioned.

As examples of suitable antioxidants, sulfides, ascorbates and the like can be mentioned.

As examples of suitable coloring agents, water-soluble food tar colors (e.g., food colors such as Food Red Nos. 2 and 3, Food Yellow Nos. 4 and 5, and Food Blue Nos. 1 and 2), water-insoluble lake pigments (e.g., aluminum salts of the aforementioned water-soluble food tar colors and the like), natural pigments (e.g., β-carotene, chlorophyll, red iron oxide and the like) and the like can be mentioned.

As examples of suitable sweeteners, sodium saccharate, dipotassium glycyrrhizinate, aspartame, stevia and the like can be mentioned.

As examples of dosage forms of the aforementioned pharmaceutical composition, oral formulations such as tablets, capsules (including soft capsules and microcapsules), granules, powders, syrups, emulsions and suspensions; non-oral formulations such as injections (e.g., subcutaneous injections, intravenous injections, intramuscular injections, intraperitoneal injections and the like), external formulations (e.g., nasal preparations, transdermal preparations, ointments and the like), suppositories (e.g., rectal suppositories, vaginal suppositories and the like), pellets, drops, sustained-release preparations (e.g., sustained-release microcapsules and the like) and the like can be mentioned; these can be safely administered orally or non-orally.

The pharmaceutical composition can be produced by a method conventionally used in the field of pharmaceutical preparation making, for example, a method described in the Japanese Pharmacopoeia and the like. A specific method of producing a preparation is hereinafter described in detail. The content of the compound obtained by the screening method of the present invention in the pharmaceutical composition varies depending on the dosage form, the dose of the compound and the like; and is, for example, from about 0.1 to 100% by weight.

For example, an oral formulation is produced by adding to an active ingredient an excipient (e.g., lactose, saccharose, starch, D-mannitol and the like), a disintegrant (e.g., calcium carboxymethyl cellulose and the like), a binder (e.g., gelatinized starch, gum arabic, carboxymethyl cellulose, hydroxypropyl cellulose, polyvinyl pyrrolidone and the like), a lubricant (e.g., talc, magnesium stearate, polyethylene glycol 6000 and the like) and the like, compression-molding the resultant mixture, and subsequently, as required, coating the resulting material with a coating base by a method known per se for the purpose of taste masking, enteric solubility or sustained release.

As examples of the coating base, a sugar-coating base, a water-soluble film coating base, an enteric film coating base, a sustained-release film coating base and the like can be mentioned.

As the sugar-coating base, saccharose is used, which may be used in combination with one species or two or more species selected from among talc, precipitated calcium carbonate, gelatin, gum arabic, pullulan, carnauba wax and the like.

As examples of the water-soluble film coating base, cellulose polymers such as hydroxypropyl cellulose, hydroxypropylmethyl cellulose, hydroxyethyl cellulose and methylhydroxyethyl cellulose; synthetic polymers such as polyvinylacetal diethylanimoacetate, aminoalkylmethacrylate copolymer E [Eudragit-E (trade name), Rohm Pharma Corp.] and polyvinyl pyrrolidone; polysaccharides such as pullulan; and the like can be mentioned.

As examples of the enteric film coating base, cellulose polymers such as hydroxypropylmethyl cellulose phthalate, hydroxypropylmethyl cellulose acetate succinate, carboxymethylethyl cellulose, and cellulose acetate phthalate; acrylic polymers such as Methacrylic Acid Copolymer L [Eudragit-L (trade name), Rohm Pharma Corp.], Methacrylic Acid Copolymer LD [Eudragit-L-30D55 (trade name), Rohm Pharma Corp.], and Methacrylic Acid Copolymer S [Eudragit-S (trade name), Rohm Pharma Corp.]; natural substances such as shellac, and the like can be mentioned.

As examples of the sustained-release film coating base, cellulose polymers such as ethyl cellulose; acrylic polymers such as aminoalkyl methacrylate copolymer RS [Eudragit-RS (trade name), Rohm Pharma Corp.], and an ethyl acrylate-methylmethacrylate copolymer suspension [Eudragit-NE (trade name), Rohm Pharma Corp.]; and the like can be mentioned.

The above-mentioned coating bases may also be used in a mixture of two or more kinds thereof in a suitable ratio. Also, during coating, a shading agent, for example, titanium oxide or iron sesquioxide, may be used.

An injection is produced by dissolving, suspending or emulsifying an active ingredient in an aqueous solvent (e.g., distilled water, physiological saline, Ringer's solution and the like), an oily solvent (e.g., vegetable oils such as olive oil, sesame oil, cottonseed oil and corn oil, propylene glycol, and the like), or the like, along with a dispersing agent (e.g., polysorbate 80, polyoxyethylene hydrogenated castor oil 60, polyethylene glycol, carboxymethyl cellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like), and the like. At this time, if desired, additives such as a solubilizing agent (e.g., sodium salicylate, sodium acetate and the like), a stabilizer (e.g., human serum albumin and the like), a soothing agent (e.g., benzyl alcohol and the like) and the like may also be used. An injection solution is normally packed in an appropriate ampule.

Because the preparation thus obtained is safe and of low toxicity, it can be administered orally or non-orally to, for example, a mammal (for example, human, mouse, rat, rabbit, sheep, swine, bovine, horse, bird, cat, dog, monkey, chimpanzee and the like).

The dosage of the prophylactic or therapeutic agent for a disease associated with the protein of the present invention or a salt thereof varies depending on target disease, subject of administration, route of administration and the like; in an adult patient suffered from a renal disease (body weight 60 kg), for example, the dosage is about 0.1 to 100 mg,
preferably about 1.0 to 50 mg, more preferably about 1.0 to 20 mg, per day, based on the active ingredient compound obtained by the screening method of the present invention.

The present invention further relates to a screening method for a prophylactic or therapeutic substance for a disease associated with the protein of the present invention or a salt thereof, which comprises using a polynucleotide comprising the base sequence that encodes the protein of the present invention or a partial peptide thereof (hereinafter also abbreviated as "the polynucleotide of the present invention").

Here, as the disease associated with the protein of the present invention or a salt thereof, the same as those mentioned for the screening method using the protein or the like of the present invention can be mentioned, but it is preferably a renal disease or diabetes, more preferably diabetic nephropathy.

Although the polynucleotide of the present invention may be any of DNA, RNA and DNA/RNA chimera, as long as it contains the base sequence that encodes the protein of the present invention or a partial peptide thereof, it is preferably DNA. These may be double-stranded or single-stranded. In the case of a double-stranded polynucleotide, it may be double-stranded DNA, double-stranded RNA or a DNA:RNA hybrid. In the case of a single-stranded polynucleotide, it may be a sense strand (i.e., coding strand) or an antisense strand (i.e., non-coding strand). As the DNA that encodes the protein of the present invention or a partial peptide thereof, those mentioned above for the method of producing the protein or the like of the present invention by gene engineering techniques can be mentioned.

The screening method using the polynucleotide of the present invention is conducted by, for example, measuring and comparing the amount of mRNA that encodes the protein of the present invention or a partial peptide thereof using the polynucleotide of the present invention between a case wherein a cell having an ability to produce the protein or the like of the present invention is cultivated in the absence of a test substance, and a case wherein the cell is cultivated in the presence of the test substance, and the like.

Here, as the cell having an ability to produce the protein or the like of the present invention, the method of cultivating the cell, and the test substance, the same as those mentioned for the aforementioned screening method using the protein or the like of the present invention can be mentioned.

The amount of mRNA that encodes the protein of the present invention or a partial peptide thereof can be quantified according to a publicly known method, for example, a method described in Molecular Cloning, 2nd edition (ibidem), or a method based thereon. For example, it can be quantified by extracting total RNA or polyA (+) RNA from a culture of a cell having an ability to produce the protein or the like of the present invention by a conventional method, conducting Northern hybridization with the polynucleotide of the present invention, for example, a polynucleotide comprising the entire or a portion of the base sequence shown by SEQ ID NO:1, as a probe, or conducting the RT-PCR method with a pair of oligonucleotides comprising a portion of the base sequence shown by SEQ ID NO:1 as primers.

For example, a test substance that increases the amount of mRNA that encodes the protein of the present invention or a partial peptide thereof by about 20% or more, preferably 30% or more, more preferably about 50% or more, as a substance for promoting the expression of the gene that encodes the protein of the present invention; and a test substance that decreases the amount of mRNA that encodes the protein of the present invention by about 20% or more, preferably 30% or more, more preferably about 50% or more, as a substance for inhibiting the expression of the gene that encodes the protein of the present invention, can be selected.

The present invention also relates to a screening kit that can be used for the above-described screening method using the polynucleotide of the present invention. The kit can include as its constituents at least one of, preferably both of (a) the above-described cell having an ability to produce the protein or the like of the present invention and (b) a reagent capable of detecting the expression of the gene that encodes the protein or the like of the present invention, for example, a polynucleotide capable of hybridizing to the mRNA that encodes the protein or the like of the present invention under highly stringent conditions. As required, the kit may further include as its constituents other reagents and instruments necessary or preferable for the conduct of the above-described screening method using the polynucleotide of the present invention.

The prophylactic or therapeutic substance for a disease associated with the protein of the present invention or a salt thereof, obtained by the screening method using the polynucleotide of the present invention, may be any of a peptide, a protein, a non-peptide compound, a synthetic compound, a fermentation product, a cell extract, a plant extract, an animal tissue extract, plasma and the like. These may have formed a salt, and as specific examples of the salt, the same as the aforementioned salts of the protein of the present invention can be mentioned.

The prophylactic or therapeutic substance (compound) for a disease associated with the protein of the present invention or a salt thereof, obtained by the screening method, can be used as a prophylactic or therapeutic agent for a disease associated with the protein of the present invention or a salt thereof after it is mixed with a pharmacologically acceptable carrier as required to yield a pharmaceutical composition.

Here, as the pharmacologically acceptable carrier, the same as those for the prophylactic or therapeutic substance for a disease associated with the protein of the present invention or a salt thereof, obtained by the screening method using the protein or the like of the present invention, can be mentioned.

The pharmaceutical composition can be produced in the same manner as the prophylactic or therapeutic substance for a disease associated with the protein of the present invention or a salt thereof, obtained by the screening method using the protein or the like of the present invention.

Because the preparation thus obtained is safe and of low toxicity, it can be administered orally or non-orally to, for example a mammal (for example, human, mouse, rat, rabbit, sheep, swine, bovine, horse, bird, cat, dog, monkey, chimpanzee, and the like).

The dosage of the prophylactic or therapeutic agent for a disease associated with the protein of the present invention or a salt thereof varies depending on target disease, subject of administration, route of administration and the like; in an adult patient suffered from a renal disease (body weight 60 kg), for example, the dosage is about 0.1 to 100 mg, preferably about 1.0 to 50 mg, more preferably about 1.0 to 20 mg, per day, based on the active ingredient compound obtained by the screening method of the present invention.

A prophylactic or therapeutic substance for a disease associated with the protein of the present invention or a salt thereof can also be screened for by detecting the promoter activity of the gene that encodes the protein.

In a cell or non-human mammal wherein DNA that encodes the protein of the present invention is substituted by a reporter gene, the activity of the promoter can be detected by confirming the expression of the protein encoded by the reporter gene after treatment with a test substance or administration of a test substance because the reporter gene is present under the control of the promoter of the gene that encodes the protein of the present invention.

In addition, the activity of the promoter can also be detected in a cell or non-human mammal having a vector prepared by joining the transcription regulatory region of the gene that encodes the protein of the present invention and a reporter gene.

Here, as examples of the reporter gene, the β-galactosidase gene (lacZ), the soluble alkaline phosphatase gene, the luciferase gene and the like can be mentioned.

For example, when a portion of the DNA region that encodes the protein of the present invention has been replaced by the *Escherichia coli*-derived β-galactosidase gene (lacZ), β-galactosidase is expressed, in place of the protein of the present invention, in tissues where the protein of the present invention is expressed originally. Therefore, the expression state of the protein of the present invention can be conveniently confirmed by, for example, staining with a reagent that can serve as a substrate for β-galactosidase, like 5-bromo-4-chloro-3-indolyl-p-galactopyranoside (X-gal). Specifically, the expression state of the protein of the present invention in a cell or tissue can be confirmed by fixing a cell or tissue section with glutaraldehyde and the like, washing the cell or section with phosphate-buffered saline (PBS), carrying out the reaction with a staining solution containing X-gal at room temperature or at nearly 37°C for about 30 minutes to 1 hour, washing the tissue specimen with 1mM EDTA/PBS solution to stop the β-galactosidase reaction, and examining the color developed. Also, the mRNA that encodes lacZ may be detected according to a conventional method.

Because a compound that promotes or inhibits the promoter activity of the gene that encodes the protein of the present invention is capable of regulating the production and activity of the protein or a salt thereof, it is useful as a prophylactic or therapeutic agent for a disease associated with the protein of the present invention or a salt thereof.

Because the protein of the present invention or the mRNA that encodes the same has its expression increased in, for example, diabetes, renal diseases (e.g., diabetic nephropathy) and the like, it is useful as a marker for early diagnosis, symptom severity determination, and disease progression prediction in the disease. Therefore, an antibody against the protein or the like of the present invention and the above-described polynucleotide of the present invention are useful as diagnostic reagents for a disease associated with the protein of the present invention or a salt thereof. Furthermore, because the antibody binds to the protein of the present invention or a salt thereof to inactivate (neutralize) the same, and also because an antisense polynucleotide of the DNA that encodes the protein of the present invention hybridizes to the mRNA that encodes the protein of the present invention to inhibit its translation into the protein, they are useful as prophylactic or therapeutic agents for a disease associated with the protein of the present invention or a salt thereof, as with the compounds obtained by the screening method of the present invention.

Accordingly, the present invention also relates to a prophylactic or therapeutic agent for a disease associated with the protein of the present invention or a salt thereof, which contains an antibody against the protein or the like of the present invention. Here, as the disease associated with the protein of the present invention or a salt thereof, those mentioned to exemplify the disease characterized by an increased amount of the protein of the present invention or a salt thereof compared to normal cases, out of the diseases mentioned for the screening method using the protein or the like of the present invention, can be mentioned, but the disease is preferably a renal disease or diabetes, more preferably diabetic nephropathy.

An antibody against the protein or the like of the present invention (hereinafter also abbreviated as "the antibody of the present invention") can be produced according to a method known per se of producing an antibody or antiserum using the protein or the like as an antigen. A monoclonal antibody or polyclonal antibody against the protein or the like of the present invention can be produced, for example, as described below.

### [Preparation of monoclonal antibody]

### (a) Preparation of monoclonal antibody-producing cells

The protein or the like of the present invention, as is or along with a carrier or a diluent, is administered to a mammal at a site permitting antibody production by administration. To increase antibody productivity in this administration, complete Freund's adjuvant and incomplete Freund's adjuvant may be administered. The administration is normally conducted every 2 to 6 weeks, in a total of about 2 to 10 times. As examples of the mammal used, monkey, rabbit, dog, guinea pig, mouse, rat, sheep, goat, and chicken can be mentioned, and a mouse and a rat are preferably used.

For example, a monoclonal antibody-producing hybridoma can be prepared by selecting an individual with an antibody titer from among antigen-immunized mammals, for example, mice, collecting the spleen or a lymph node 2-5 days after final immunization, and fusing an antibody-producing cell contained therein with an allogeneic or heterogeneous myeloma cell. A measurement of antibody titer in the antiserum can be conducted by, for example, reacting the labeled protein described below and an antiserum, and thereafter measuring the activity of the labeling agent bound to the antibody. The fusion procedure can be performed according to a known method, for example, the method of Köhler and Milstein [Nature, 256, 495 (1975)]. As examples of a fusogen, polyethylene glycol (PEG), Sendai virus and the like can be mentioned, and PEG is preferably used.

As examples of the myeloma cell, mammalian myeloma cells such as NS-1, P3U1, Sp2/O and AP-1 can be mentioned, and P3U1 is preferably used. A preferable ratio of the number of antibody-producing cells (splenocytes) and number of myeloma cells used is about 1:1 to 20:1; cell fusion can be efficiently performed by adding a PEG (preferably PEG1000 to PEG6000) at concentrations of about 10 to 80%, and conducting incubation at 20 to 40°C, preferably at 30 to 37°C, for 1 to 10 minutes.

A monoclonal antibody-producing hybridoma can be screened for by, for example, a method wherein the hybridoma culture supernatant is added to a solid phase (e.g., microplate) having a protein antigen adsorbed thereto directly or along with a carrier, an anti-immunoglobulin antibody (when the cell used for cell fusion is a mouse cell, an anti-mouse immunoglobulin antibody is used) or protein A labeled with a radioactive substance, an enzyme or the like is then added, and the monoclonal antibody bound to the solid phase is detected, a method wherein the hybridoma culture supernatant is added to a solid phase having an anti-immunoglobulin antibody or protein A adsorbed thereto, a protein labeled with a radioactive substance, an enzyme or the like is added, and the monoclonal antibody bound to the solid phase is detected, and the like.

Selection of a monoclonal antibody can be conducted according to a method known per se or a method based thereon. Selection of a monoclonal antibody can normally be conducted using an animal cell culture medium supplemented with HAT (hypoxanthine, aminopterin, thymidine). As the medium for selection and breeding of a monoclonal antibody, any medium can be used, as long as the hybridoma can grow therein. For example, an RPMI 1640 medium containing 1 to 20%, preferably 10 to 20%, fetal bovine serum, a GIT medium (Wako Pure Chemical Industries, Ltd.) containing 1 to 10% fetal bovine serum or a serum-free medium for hybridoma culture (SFM-101, Nissui Pharmaceutical Co., Ltd.) and the like can be used. Cultivation temperature is normally 20 to 40°C, preferably about 37°C. Cultivation time is normally 5 days to 3 weeks, preferably 1 week to 2 weeks. Cultivation can normally be conducted under 5% carbonic acid gas. The antibody titer of the hybridoma culture supernatant can be measured in the same manner as the above-described measurement of the antibody titer in the antiserum.

The thus-obtained monoclonal antibody can be separated and purified according to a method known per se, for example, a method of immunoglobulin separation and purification [e.g., salting-out method, alcohol precipitation method, isoelectric point precipitation method, electrophoresis method, adsorption and desorption method using an ion exchanger (e.g., DEAE), ultracentrifugation method, gel filtration method, specific purification method wherein only the antibody is collected using an antigen-binding solid phase or an active adsorbent such as protein A or protein G, and its bond is dissociated to yield the antibody].

### [Preparation of polyclonal antibody]

A polyclonal antibody against the protein or the like of the present invention can be produced according to a method known per se. For example, the polyclonal antibody can be produced by immunizing a mammal with an immune antigen (protein antigen) as is or a complex thereof with a carrier protein in the same manner as the above-described method of monoclonal antibody production, collecting the antibody-containing product of the present invention from the immunized animal, and separating and purifying the antibody.

Regarding the complex of an immune antigen and carrier protein used to immunize a mammal, any kind of carrier protein can be crosslinked at any mixing ratio of carrier and hapten, as long as an antibody against the carrier-crosslinked immunized hapten is efficiently produced; for example, a method
wherein bovine serum albumin, bovine thyroglobulin, hemocyanin or the like is coupled at a ratio of about 0.1 to 20, preferably about 1 to 5, parts by weight per 1 part by weight of hapten, can be used.

For coupling of a hapten and a carrier, various condensing agents, for example, active ester reagents containing glutaraldehyde, carbodiimide, a maleimide active ester, a thiol group or a dithiopyridyl group, and the like can be used.

The condensation product, as is or along with a carrier or a diluent, is administered to a mammal at a site permitting antibody production. To increase antibody productivity in this administration, complete Freund's adjuvant and incomplete Freund's adjuvant may be administered. The administration is normally conducted every 2 to 6 weeks, in a total of about 3 to 10 times.

A polyclonal antibody can be collected from blood, ascites fluid and the like, preferably blood, of a mammal immunized by the above-described method.

A measurement of the polyclonal antibody titer in the antiserum can be conducted in the same manner as the above-described measurement of antibody titer in the antiserum. Separation and purification of the polyclonal antibody can be conducted according to the same immunoglobulin separation and purification method as the above-described monoclonal antibody separation and purification.

Although the prophylactic or therapeutic agent for a disease associated with the protein of the present invention or a salt thereof, which contains the antibody of the present invention, may be the antibody of the present invention as is, it is preferably a pharmaceutical composition prepared by mixing the antibody and a pharmacologically acceptable carrier. Here, as the pharmacologically acceptable carrier, the same as those mentioned for the aforementioned "prophylactic or therapeutic substance for a disease associated with the protein of the present invention or a salt thereof" can be mentioned.

The pharmaceutical composition can be produced in the same manner as the aforementioned "prophylactic or therapeutic substance for a disease associated with the protein of the present invention or a salt thereof".

Because the preparation thus obtained is safe and of low toxicity, it can be administered orally or non-orally to, for example, a mammal (for example, human, mouse, rat, rabbit, sheep, swine, bovine, horse, bird, cat, dog, monkey, chimpanzee, and the like).

The dosage of the prophylactic or therapeutic agent varies depending on target disease, subject of administration, route of administration and the like; in an adult patient suffered from a renal disease (body weight 60 kg), for example, the dosage is about 0.1 to 100 mg, preferably about 1.0 to 50 mg, more preferably about 1.0 to 20 mg, per day, based on the active ingredient antibody of the present invention.

The present invention also relates to a prophylactic or therapeutic agent for a disease associated with the protein of the present invention or a salt thereof, which contains a polynucleotide comprising a base sequence complementary to the base sequence that encodes the protein or a partial peptide thereof. Here, as the disease associated with the protein of the present invention or a salt thereof, those mentioned as diseases characterized by an increased amount of the protein of the present invention or a salt thereof compared to normal cases, out of the diseases mentioned for the screening method using the protein or the like of the present invention, can be mentioned, but it is preferably a renal disease or diabetes, more preferably diabetic nephropathy.

As the polynucleotide having a base sequence complementary to the base sequence that encodes the protein of the present invention or a partial peptide thereof (hereinafter also abbreviated as "the antisense polynucleotide of the present invention"), any polynucleotide can be mentioned, as long as it has a base sequence completely complementary, or substantially complementary, to the base sequence that encodes the protein of the present invention or a partial peptide thereof, and acts to suppress the translation of the protein from the RNA that encodes the protein of the present invention. As the "substantially complementary base sequence", a base sequence capable of hybridizing to the base sequence that encodes the protein of the present invention or a partial peptide thereof under the physiological conditions for the cell that expresses the protein, more specifically, a base sequence having a homology of about 70% or more, preferably about 80% or more, more preferably about 90% or more, and most preferably about 95% or more, to the complementary strand of the base sequence that encodes the protein of the present invention or a partial peptide thereof, and the like can be mentioned.

The antisense polynucleotide of the present invention can be designed and synthesized on the basis of information on the cloned or determined base sequence of the polynucleotide of the present invention. Such a polynucleotide is capable of inhibiting the replication or expression of the gene that encodes the protein of the present invention. Hence, the antisense polynucleotide of the present invention is capable of hybridizing to the RNA transcribed from the gene that encodes the protein of the present invention, and capable of inhibiting the synthesis (processing) or function (translation into protein) of mRNA.

The target region of the antisense polynucleotide of the present invention is not subject to limitation as to the length thereof, as long as hybridization of the antisense polynucleotide results in the inhibition of the translation of the protein of the present invention, and can be the entire sequence or a partial sequence of the RNA that encodes the protein of the present invention; a partial sequence of about 15 bases for the shortest, and the entire sequence of the mRNA or initial transcription product for the longest, can be mentioned. Considering the ease of synthesis and the issue of antigenicity, an oligonucleotide comprising about 15 to about 30 bases is preferred, which, however, is not to be construed as limiting. Specifically, although the 5'-end hairpin loop, the 5'-end 6-base-pair repeat, the 5'-end untranslated region, the polypeptide translation initiation codon, the protein-coding region, the ORF translation initiation codon, the 3'-end untranslated region, the 3'-end palindrome region, and the 3'- end hairpin loop of the gene that encodes the protein of the present invention can be selected as the target region, any region within the gene can be selected as the target. For example, it is also preferable that the intron portion of the gene be the target region.

Furthermore, the antisense polynucleotide of the present invention may be one capable of not only hybridizing to the mRNA that encodes the protein of the present invention or the initial transcription product thereof to inhibit the translation to the protein, but also binding to the gene that encodes the protein of the present invention, which is double-stranded DNA, to form a triple strand (triplex) and inhibit the transcription of RNA.

As the antisense polynucleotide, a deoxyribonucleotide containing 2-deoxy-D-ribose, a ribonucleotide containing D-ribose, another type of nucleotide that is an N-glycoside of the purine or pyrimidine base, or another polymer having a non-nucleotide backbone (for example, commercially available protein nucleic acids and synthetic sequence specific nucleic acid polymers) or another polymer having a special bond (however, this polymer contains a nucleotide having a configuration that allows base pairing or base attachment as found in DNA and RNA) and the like can be mentioned. These may be double-stranded DNAs, single-stranded DNAs, double-stranded RNAs or single-stranded RNAs, or DNA:RNA hybrids, and may also non-modified polynucleotides (or non-modified oligonucleotides), those having a known modification added thereto, for example, those with a marker known in the relevant field, those with a cap, those methylated, those having 1 or more naturally occurring nucleotides substituted by analogues, those modified with an intramolecular nucleotide, for example, those having a non-charge bond (for example, methylphosphonate, phospho triester, phosphoramidate, carbamate and the like), those having a charged bond or a sulfur-containing bond (for example, phosphorothioate, phosphorodithioate and the like), for example, those having a side chain group of a protein (nuclease, nuclease inhibitor, toxin, antibody, signal peptide, poly-L-lysine and the like), or a sugar (for example, monosaccharide and the like) and the like, those having an intercalating compound (for example, acridine, psoralen and the like), those containing a chelate compound (for example, metals, radioactive metals, boron, oxidizing metals and the like), or those containing an alkylating agent, those having a modified bond (for example, α anomer type nucleic acid and the like). Here, "nucleoside", "nucleotide" and "nucleic acid" may include not only those containing the purine and pyrimidine bases, but also those containing another modified heterocyclic base. These modified products may contain a methylated purine and pyrimidine, an acylated purine and pyrimidine, or another heterocycle. The modified nucleotide and the modified nucleotide may also have their sugar portion modified by, for example, substitution of 1 or more hydroxyl groups by a halogen, an aliphatic group and the like, or conversion to a functional group such as an ether or an amine.

The antisense polynucleotide is RNA, DNA or a modified nucleic acid (RNA, DNA). As specific examples of the modified nucleic acid, sulfur derivatives and thiophosphate derivatives of nucleic acids, and those resistant to the decomposition like polynucleosideamide or oligonucleosideamide can be mentioned, which, however, are not to be construed as limiting. The antisense nucleic acid of the present invention can preferably be designed to accomplish one of the following purposes: to make the antisense nucleic acid more stable in the cell, to increase the cell permeability of the antisense nucleic acid, to increase the affinity for the desired sense strand, and to reduce the toxicity, if any, of the antisense nucleic acid. Many such modifications are known in the relevant field, and are disclosed in, for example, J. Kawakami et al., Pharm Tech Japan, Vol. 8, pp. 247, 1992; Vol. 8, pp. 395, 1992; S. T. Crooke et al. ed., Antisense Research and Applications, CRC Press, 1993 and the like.

The antisense polynucleotide may be altered, and may contain an modified sugar, base or bond, and can be supplied in a special form like liposome or microspheres, can be applied for gene therapy, and can be given in an adduct form. As such an adduct form used, a polycation like polylysine, which acts to neutralize the charge of the phosphate backbone, and a hydrophobic compound like a lipid that enhances the interaction with cell membrane or increases nucleic acid uptake (for example, phospholipid, cholesterol and the like) can be mentioned. As lipids preferred for addition, cholesterol and derivatives thereof (for example, cholesterylchloroformate, cholic acid and the like) can be mentioned. These can be attached to the 3' end or the 5' end of nucleic acid, and can be attached via a base, a sugar or an intramolecular nucleoside bond. As other groups, a capping group specifically arranged at the 3' end or 5' end of nucleic acid to prevent degradation by a nuclease such as exonuclease or RNase can be mentioned. As such a capping group, hydroxyl group protecting groups known in the relevant field, including glycols such as polyethylene glycol and tetraethylene glycol can be mentioned, which, however, are not to be construed as limiting.

A ribozyme capable of specifically cleaving the mRNA or the initial transcription product that encodes the protein of the present invention within the coding region (including the intron portion in the case of the initial transcription product) can also be encompassed in the antisense polynucleotide of the present invention. "Ribozyme" refers to RNA possessing an enzyme activity to cleave a nucleic acid, and is herein understood to be used as a concept encompassing DNA, as long as sequence-specific nucleic acid cleavage activity is possessed, since it has recently been found that oligo DNA having the base sequence of the enzyme activity portion also possesses nucleic acid cleavage activity. One of the most versatile ribozymes is self-splicing RNA found in infectious RNAs such as viroid and virusoid, and the hammerhead type, the hairpin type and the like are known. The hammerhead type exhibits enzyme activity with about 40 bases in length, and it is possible to specifically cleave the target mRNA by making several bases at both ends adjoining to the hammerhead structure portion (about 10 bases in total) to be a sequence complementary to the desired cleavage site of the mRNA. Because this type of ribozymes has RNA only as the substrate, it offers an additional advantage of non-attack of genomic DNA. Provided that the mRNA corresponding to "the polynucleotide of the present invention" takes a double-stranded structure by itself, the target sequence can be made single-stranded, using a hybrid ribozyme prepared by joining an RNA motif derived from a viral nucleic acid that can specifically bind to RNA helicase [Proc. Natl. Acad. Sci. USA, 98 (10): 5572-5577 (2001)]. Furthermore, when the ribozyme is used in the form of an expression vector containing the DNA that encodes it, the ribozyme may be a hybrid ribozyme prepared by further joining a sequence modified from the tRNA to promote the migration of the transcription product to cytoplasm [Nucleic Acids Res., 29(13): 2780-2788 (2001)].

A double-stranded oligo RNA complementary to a partial sequence (including the intron portion in the case of the initial transcription product) within the coding region of the mRNA or the initial transcription product that encodes the protein of the present invention can also be encompassed in the antisense polynucleotide of the present invention. RNA interference (RNAi), a phenomenon in which introducing short double-stranded RNA in a cell leads to the decomposition of mRNA complementary to the RNA, has been known to occur in nematodes, insects, plants and the like, and since this phenomenon has recently been found to occur in mammalian cells as well [Nature, 411(6836): 494-498 (2001)], it is attracting attention for technology to replace ribozymes.

The antisense oligonucleotide and ribozyme of the present invention can be prepared by determining the target region of the mRNA or initial transcription product on the basis of information on the cDNA sequence or genomic DNA sequence that encodes the protein of the present invention, and synthesizing a sequence complementary thereto using a commercially available DNA/RNA synthesizer (Applied Biosystems, Beckman Instruments, and the like). Double-stranded oligo RNA possessing RNAi activity can be prepared by synthesizing each of a sense strand and an antisense strand using a DNA/RNA synthesizer, denaturing the strands in an appropriate annealing buffer solution at, for example, about 90 to about 95°C for about 1 minute, and then annealing the strands at about 30 to about 70°C for about 1 to about 8 hours. It is also possible to prepare a longer double-stranded polynucleotide by synthesizing complementary oligonucleotide strands in alternative overlaps, annealing the strands, and ligating the strands using ligase.

Although the prophylactic or therapeutic agent for a disease associated with the protein of the present invention or a salt thereof, which comprises the antisense polynucleotide of the present invention, may be the antisense polynucleotide of the present invention as is, it is preferably a pharmaceutical composition prepared by mixing the antisense polynucleotide with a pharmacologically acceptable carrier. The antisense polynucleotide can be formulated and administered orally or non-orally to a mammal (for example, human, mouse, rat, rabbit, sheep, swine, bovine, horse, bird, cat, dog, monkey, chimpanzee, and the like) in the same manner as the prophylactic or therapeutic substance for a disease associated with the protein of the present invention or a salt thereof, obtained by the screening method using the protein or the like of the present invention. The antisense polynucleotide can also be administered after insertion to an appropriate vector, for example, retrovirus vector, adenovirus vector, adenovirus-associated virus vector and the like.

The antisense polynucleotide can be administered using a gene gun or a catheter like a hydrogel catheter, and can also be administered locally into the trachea as an inhalant after conversion to an aerosol.

The dosage of the antisense polynucleotide varies depending on target disease, subject of administration, route of administration and the like; in an adult patient suffered from a renal disease (body weight 60 kg), for example, the dosage is about 0.1 to 100 mg, preferably about 1.0 to 50 mg, more preferably about 1.0 to 20 mg, per day.

Furthermore, the antisense polynucleotide of the present invention can also be used as a diagnostic oligonucleotide probe to examine the presence and the expression manner of a polynucleotide that encodes the protein of the present invention in a tissue or cell.

The present invention also relates to a diagnostic reagent for a disease associated with the protein of the present invention or a salt thereof, which comprises the above-described "polynucleotide of the present invention". For example, because it is possible to detect an abnormality (genetic abnormality) in the DNA or mRNA that encodes the protein of the present invention in a mammal (for example, human, rat, mouse, guinea pig, rabbit, bird, sheep, swine, bovine, horse, cat, dog, monkey, chimpanzee, and the like) using the polynucleotide of the present invention as a probe, the polynucleotide is useful as, for example, a genetic diagnostic reagent for damage, mutation or decreased expression of the DNA or mRNA, increased expression or overexpression of the DNA or mRNA, and the like.

The above-described genetic diagnosis using the polynucleotide of the present invention can be performed by, for example, a method known per se, such as Northern hybridization and the PCR-SSCP method (Genomics, Vol. 5, pp. 874 to 879 (1989), Proceedings of the National Academy of Sciences of the United States of America, Vol. 86, pp. 2766 to 2770 (1989)) and the like.

For example, if overexpression or decreased expression is detected by Northern hybridization, or if a DNA mutation is detected by the PCR-SSCP method, the test animal could be diagnosed as being likely to have a disease associated with the protein of the present invention or a salt thereof, for example, diabetes, a renal disease and the like.

The present invention also relates to a diagnostic reagent for a disease associated with the protein of the present invention or a salt thereof, which contains the above-described "antibody of the present invention".

Accordingly, the present invention provides:
(i) a method of diagnosing a disease associated with the protein of the present invention or a salt thereof, which comprises competitively reacting the antibody of the present invention, a test solution, and the protein or the like of the present invention labeled, and determining the ratio of the labeled protein or the like of the present invention bound to the antibody, to quantity the protein of the present invention or a salt thereof in the test solution, and
(ii) a method of diagnosing a disease associated with the protein of the present invention or a salt thereof, which comprises reacting a test solution, an antibody of the present invention insolubilized on a carrier, and another antibody of the present invention labeled, simultaneously or serially, and then measuring the activity of the labeling agent on the insolubilizing carrier to quantity the protein of the present invention or a salt thereof in the test solution.

In the quantitation of (ii) above, if one of the two antibodies is an antibody that recognizes an N-terminal portion of the protein or the like of the present invention, the other antibody is desirably an antibody that recognizes another portion, for example, a C-terminal portion, of the protein or the like of the present invention.

In addition to the quantitation of the protein or the like of the present invention using a monoclonal antibody against the protein, detection by tissue staining and the like can also be conducted. For these purposes, the antibody molecule itself may be used, and the F(ab')₂, Fab' or Fab fraction of the antibody molecule may also be used.

The quantitation of the protein of the present invention or a salt thereof using the antibody of the present invention is not subject to limitation, and any method of measurement can be used, as long as it is a measurement method wherein the amount of antibody, antigen or antibody-antigen complex corresponding to the amount of antigen in the test solution is detected by a chemical or physical means and is applied to a standard curve generated using standard solutions containing known amounts of antigen. For example, nephelometry, the competitive method, the immunometric method and the sandwich method are preferably used; it is particularly preferable, in terms of sensitivity and specificity, to use the sandwich method described below.

As examples of the labeling agent used for the measurement method using a labeled substance, a radioisotope, an enzyme, a fluorescent substance, a luminescent substance and the like can be used. As examples of the radioisotope, [¹²⁵I], [¹³¹I] , [³H] , [¹⁴C] and the like can be used. As the above-described enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-(strepto)avidin system can also be used for binding of an antibody or an antigen and a labeling agent.

In insolubilizing the antigen or antibody, physical adsorption may be used, and a method based on a chemical bond conventionally used to insolubilize or immobilize a protein or the like, may also be used. As the carrier, insoluble polysaccharides such as agarose, dextran and cellulose, synthetic resins such as polystyrene, polyacrylamide and silicone, glass and the like can be mentioned.

In the sandwich method, the amount of protein of the present invention or a salt thereof in a test solution can be quantified by reacting the test solution to a monoclonal antibody of the present invention insolubilized (primary reaction) and further reacting to another monoclonal antibody of the present invention labeled (secondary reaction), and thereafter measuring the activity of the labeling agent on the insolubilizing carrier. The primary reaction and the secondary reaction may be conducted in the reverse order, and may be conducted simultaneously or after a time lag. The labeling agent and the method of insolubilization can be based on those described above. Also, in the immunoassay by the sandwich method, the antibody used as the antibody for a solid phase or the antibody for labeling needs not always be one kind; a mixture of two kinds or more of antibodies may be used for the purposes of measurement sensitivity improvement and the like.

In the above-described measurement of the protein of the present invention or a salt thereof by the sandwich method, the monoclonal antibodies of the present invention used in the primary reaction and the secondary reaction are preferably antibodies having mutually different sites for binding of the protein of the present invention. Accordingly, regarding the antibodies used for the primary reaction and the secondary reaction, provided that the antibody used for the secondary reaction recognizes a C-terminal portion of the protein of the present invention, for example, the antibody used for the primary reaction is preferably an antibody that recognizes a site other than the C-terminal portion, for example, an N-terminal portion.

The monoclonal antibody of the present invention can be used for a measurement system other than the sandwich method, for example, the competitive method, the immunometric method or nephelometry and the like.

In the competitive method, the antigen and the labeled antigen in the test solution are competitively reacted with the antibody, after which the unreacted labeled antigen (F) and the antibody-bound labeled antigen (B) are separated (B/F separation), the amount labeled of either B or F is measured, and the amount of antigen in the test solution is quantified. For this reaction method, the liquid phase method, wherein a soluble antibody is used as the antibody and B/F separation is conducted using polyethylene glycol, a second antibody against the above-described antibody, and the like, and the solid phase immobilization method, wherein a solid-phase-immobilized antibody is used as the first antibody or the first antibody used is a soluble one and a solid-phase-immobilized antibody is used as the second antibody, can be used.

In the immunometric method, the antigen and the solid phase-immobilized antigen in the test solution are competitively reacted to a given amount of labeled antibody, after which the solid phase and the liquid phase are separated, or the antigen in the test solution and an excess amount of labeled antibody are reacted, a solid-phase-immobilized antigen is then added to bind the unreacted labeled antibody to the solid phase, after which the solid phase and the liquid phase are separated. Next, the amount labeled in either phase is measured to quantify the antigen in the test solution.

Also, in nephelometry, the amount of insoluble precipitate resulting from an antigen-antibody reaction in the gel or in the solution is measured. Even when the amount of antigen in the test solution is small and only a small amount of precipitate is obtained, laser nephelometry, which utilizes laser scattering, and the like are preferably used.

In applying these individual immunoassays to the quantitation method of the present invention, it is unnecessary to set special conditions, procedures and the like. Making ordinary technical considerations for those skilled in the art to the ordinary conditions and procedures in each method, a measurement system for the protein of the present invention can be constructed. For details of these general technical means, compendia, books and the like can be referred to.

For example, edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.

Using the antibody of the present invention as described above, the protein of the present invention or a salt thereof can be quantified at high sensitivity.

In the above-described quantitation method using the antibody of the present invention, the concentration of the protein of the present invention or a salt thereof in a biological sample (e.g., kidney cell, pancreatic cell and the like) from a test animal as analyte is quantified; if overexpression or decreased expression of the protein is detected, the test animal could be diagnosed as being likely to suffer from a disease associated with the protein of the present invention or a salt thereof, for example, diabetes, a renal disease and the like.

The present invention further relates to a prophylactic or therapeutic agent for diabetes or a renal disease, preferably diabetic nephropathy, which contains a TSC-22 suppressant.

A TSC-22 suppressant refers to a substance capable of quantitatively and/or qualitatively suppressing TSC-22 in the body. Specifically, the TSC-22 suppressant is not subject to limitation, as long as it is a substance capable of suppressing the expression (including all levels of transcription, post-transcription regulation, translation, post-translational modification and the like) of the TSC-22 gene, or of instabilizing or deactivating the TSC-22 protein, and may be any of a peptide, a protein, a non-peptide compound, a synthetic compound, a fermentation product, a cell extract, a plant extract, an animal tissue extract, plasma and the like. These may have formed a salt, and as specific examples of the salt, the same as the aforementioned salts of the protein of the present invention can be mentioned.

The TSC-22 suppressant is preferably a substance capable of suppressing the production or expression of TSC-22 or the activity of TSC-22 in the kidney or pancreas.

A prophylactic or therapeutic agent for diabetes or a renal disease (e.g., diabetic nephropathy), which contains the TSC-22 suppressant, can be formulated according to the nature of TSC-22, in the same manner as the prophylactic or therapeutic substance for a disease associated with the protein of the present invention or a salt thereof and the antisense polynucleotide of the present invention.

Because the prophylactic or therapeutic agent is safe and of low toxicity, it can be administered orally or non-orally to, for example, a mammal (for example, human, mouse, rat, rabbit, sheep, swine, bovine, horse, bird, cat, dog, monkey, chimpanzee, and the like).

The dosage of the TSC-22 suppressant varies depending on target disease, subject of administration, route of administration and the like; in an adult patient (body weight 60 kg), for example, the dosage is about 0.1 to 100 mg, preferably about 1.0 to 50 mg, more preferably about 1.0 to 20 mg, per day.

Abbreviations for bases, amino acids and the like used herein are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
- DNA:: Deoxyribonucleic acid
- cDNA:: Complementary deoxyribonucleic acid
- A:: Adenine
- T:: Thymine
- G:: Guanine
- C:: Cytosine
- RNA:: Ribonucleic acid
- mRNA:: Messenger ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP:: Adenosine triphosphate
- EDTA:: Ethylenediaminetetraacetic acid
- SDS:: Sodium dodecyl sulfate
- Gly:: Glycine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn:: Asparagine
- Gln:: Glutamine
- pGlu:: Pyrroglutamine
- Sec:: Selenocysteine

The sequence identification numbers in the sequence listing given herein show the following sequences.
[SEQ ID NO:1]
Shows the base sequence of the protein-coding region of human TSC-22 cDNA.
[SEQ ID NO:2]
Shows the amino acid sequence of the human TSC-22 protein.
[SEQ ID NO:3]
Shows the base sequence of the DNA used as a sense probe in Example 4.

The present invention is hereinafter described in more detail by means of the following examples, which, however, are not to be construed as limiting the present invention.

### Example 1: Increases in TSC-22 mRNA expression in the kidneys of Wistar fatty rats

Using male Wistar fatty rats (13, 22 and 40 weeks of age; TAKEDA RABICS), which have non-insulin-dependent diabetes (NIDDM) and spontaneously develop diabetic nephropathy (DN), and male Wistar lean rats at the same weeks of age (TAKEDA RABICS) which are normal control rats, 24-hour urine pooling and tail vein blood drawing were conducted, and urinary albumin excretion, plasma glucose concentrations and plasma insulin concentrations were measured. Urinary albumin excretion was measured using the A/G B Test Wako (Wako Pure Chemical Industries, Ltd.), and plasma glucose concentrations were measured using Synchron CX5 Delta (Beckman Coulter). Insulin concentrations were measured by the RIA method (Shionogi & Co., Ltd.). Kidneys were collected from five rats in each group and stored at -80°C. After the sample was disrupted, total RNA was extracted. A primer and a fluorescent probe were prepared on the basis of reported mRNA sequences (Endocrinology, 134(3): 1205-1212 (1994)), and various mRNA expression levels were measured by the real-time quantitative RT-PCR method using ABI PRISM 7700 (Applied Biosystems). The results are shown in Tables 1 and 2.

**Table 1.**

| Urinary albumin excretion, plasma glucose concentrations and plasma insulin concentrations | | | | | | |
|---|---|---|---|---|---|---|
| | Wistar lean rats | | | Wistar fatty rats | | |
| Weeks of age | 13 | 22 | 40 | 13 | 22 | 40 |
| Urinary albumin excretion (mg/day) | 5.1 | 4.0 | 10.4 | 6.7 | 55.7 | 182.0 |
| Plasma glucose concentrations (mg/dl) | 138.9 | 137.1 | 134.3 | 319.2 | 402.9 | 319.4 |
| Plasma insulin concentrations (µUnits/ml) | 123.0 | 125.6 | 158.7 | 1091.9 | 1019.2 | 1674.4 |
| (n=5, Mean) | | | | | | |

**Table 2.**

| mRNA expression levels of TSC-22 and TGF-β1 in the kidneys of Wistar fatty rats (Relative values with the expression level in the kidneys of Wistar lean rats at each week of age as 1) | | | |
|---|---|---|---|
| Weeks of age | 13 | 22 | 40 |
| TSC-22 | 1.0 | 1.0 | 1.6 |
| TGF-β1 | 1.1 | 1.0 | 1.7 |
| (n=5, Mean) | | | |

The Wistar fatty rats developed NIDDM at 13 weeks of age, and developed DN and showed increased urinary albumin excretion at 22 weeks of age. In the Wistar fatty rats at 40 weeks of age, increased TSC-22 mRNA expression levels were observed, and increased transforming growth factor-β1 (TGF-β1) mRNA expression levels were also observed; a significant positive correlation (r=0.80598, p=0.0049) was observed between TSC-22 mRNA and TGF-β1 mRNA expression levels.

### Example 2: Changes in TSC-22 mRNA expression in the kidneys of Zucker fatty rats

Male Zucker fatty rats (ZF rats, 18 weeks of age, Charles River Japan Inc.), which have hyperinsulinemia and spontaneously develop renal disease, were given oral administration of candesartan cilexetil (angiotensin II type 1 receptor antagonist) in suspension in 0.5% methylcellulose 100cP once daily for 9 consecutive weeks. For a control group and a normal control group, male Zucker lean rats at the same weeks of age (ZL rats, Charles River Japan Inc.) were given oral administration of 0.5% methylcellulose 100cP (vehicle) once daily for consecutive days. At 8 weeks of administration, 24-hour urine pooling was conducted, and urinary albumin excretion was measured using the A/G B Test Wako (Wako Pure Chemical Industries, Ltd.). At 9 weeks of administration, kidneys were collected and stored at -80°C. After the sample was disrupted, total RNA was extracted. A primer and a fluorescent probe were prepared on the basis of reported (ibidem) mRNA sequences, and various mRNA expression levels were measured by the real-time quantitative RT-PCR method using ABI PRISM7700 (Applied Biosystems). The results are shown in Tables 3 and 4.

**Table 3.**

| Urinary albumin excretion, blood glucose concentrations and blood insulin concentrations | | | |
|---|---|---|---|
| | ZL rats Vehicle | ZF rats Vehicle | ZF rats Candesartan cilexetil |
| Urinary albumin excretion (mg/day) | 48.5 | 401.5 | 61.7 |
| (n=8-9, Mean) | | | |

**Table 4.**

| mRNA expression levels of TSC-22 and TGF-β1 in the kidneys of Zucker fatty rats (Relative values with the expression level in the kidneys of Zucker lean rats at each week of age as 1) | | |
|---|---|---|
| | ZF rats Vehicle | ZF rats Candesartan cilexetil |
| TSC-22 | 2.4 | 1.6 |
| TGF-β1 | 2.1 | 1.2 |
| (n=8-9, Mean) | | |

In the kidneys of the Zucker fatty rats with increased urinary albumin excretion, the TSC-22 mRNA expression level increased and the mRNA expression of TGF-β1 also increased. Furthermore, when the increase in urinary albumin excretion was suppressed by administration of candesartan cilexetil, both of the above-described increases in mRNA expression levels were suppressed.

### Example 3: Increases in TSC-22 mRNA expression in the kidneys of spontaneously hypercholesterolemic rats

Using male spontaneously hypercholesterolemic rats that spontaneously develop renal disease (SHC rats, 6, 12, 20 and 26-30 weeks of age, TAKEDA RABICS) and a normal control group of male Sprague-Dawley rats at the same weeks of age (SD rats, Clea Japan, Inc.), 24-hour urine pooling and tail vein blood drawing were conducted, and urinary albumin excretion, plasma total cholesterol concentrations and blood urea nitrogen concentrations were measured. Urinary albumin excretion was measured using the A/G B Test Wako (Wako Pure Chemical Industries, Ltd.), and plasma total cholesterol concentrations and blood urea nitrogen concentrations were measured using Synchron CX5 Delta (Beckman Coulter). Kidneys were collected and stored at -80°C. After the sample was disrupted, total RNA was extracted. A primer and a fluorescent probe were prepared on the basis of reported mRNA sequences, and each mRNA expression level was measured by the real-time quantitative RT-PCR method using ABI PRISM7700 (Applied Biosystems). The results are shown in Tables 5 and 6.

**Table 5.**

| Urinary albumin excretion, plasma glucose concentrations and plasma insulin concentrations | | | | | | | | |
|---|---|---|---|---|---|---|---|---|
| | SD rats | | | | SHC rats | | | |
| Weeks of age | 6 | 12 | 20 | 26-30 | 6 | 12 | 20 | 26-30 |
| Urinary albumin excretion (mg/day) | 6.1 | 9.2 | 13.1 | 16.4 | 8.9 | 179.2 | 598.3 | 656.6 |
| Blood urine nitrogen concentrations (mg/dl) | 18.9 | 21.5 | 22.8 | 21.5 | 15.7 | 20.2 | 40.1 | 85.1 |
| (n=4-5, Mean) | | | | | | | | |

**Table 6.**

| mRNA expression levels of TSC-22 and TGF-β1 in the kidneys of SHC rats (Relative values with the expression level in the kidneys of SD rats at each week of age as 1) | | | | |
|---|---|---|---|---|
| Weeks of age | 6 | 12 | 20 | 26-30 |
| TSC-22 | 1.9 | 3.5 | 6.0 | 12.4 |
| TGF-β1 | 1.1 | 1.4 | 4.5 | 9.0 |
| (n=4-5, Mean) | | | | |

In the SHC rats at 12 weeks of age, the urinary albumin excretion had already been increased, and threafter the excretion increased over time. In the SHC rats at 20 weeks of age or more, the blood urea nitrogen concentration also increased over time. TSC-22 mRNA expression increased 1.9 fold in the SHC rats at 6 weeks of age, and thereafter the expression level increased over time. In the SHC rats, the TSC-22 mRNA expression level showed a significant positive correlation with urinary albumin excretion (r=0.67648, p=0.0015) and with blood urea nitrogen concentration (r=0.96394, p=0.0001). Increases in the TGF-β1 mRNA expression level were observed in the SHC rats at 12 weeks of age or more. Between the TSC-22 mRNA and TGF-β1 mRNA expression levels in the SHC rat, a significant positive correlation (r=0.88356, p=0.0001) was observed.

### Example 4: TSC-22 expression in the kidneys of spontaneously hypercholesterolemic SHC rats and normal SD rats - analysis by in situ hybridization -

Kidneys were collected from SD rats and SHC rats fixed by perfusion with 10% neutrally buffered formalin. The kidneys were fixed with 10% neutrally buffered formalin and embedded in paraffin to yield block specimens. Each specimen was sectioned to a thickness of 4 µm, and these sections were used for in situ hybridization (ISH) staining. Using T3 and SP6 RNA polymerase, a digoxigenin (DIG)-labeled RNA probe was prepared on the basis of the sequence shown below. DIG labeling was achieved using DIG RNA Labeling Mix (Roche). With the GAPDH (glyceraldehyde-3-phosphate-dehydrogenase) gene as positive control, ISH conditions in the rat kidney were confirmed, and ISH was conducted using a TSC-22 antisense probe and its sense probe [SEQ ID NO:3; a portion of the 3'-untranslated region (base numbers: 611-952) of rat TSC-22 mRNA (GenBank Accession Number: L25785)], anti-DIG antibody, and as a color development substrate NBT/BCIP (nitro blue tetrazolium/5-bromo-4-chloro-3-indolyl phosphate). After staining, nuclear staining was conducted using Kernechtrot.

As a result, as shown in FIG. 1, signals were observed in proximal tubules, thin-wall tubules, and epithelial cells of glomerular capsule and podocytes, and epithelial cells of distal tubules and collecting ducts, in SHC rat kidneys, with evidently greater signal intensity than in normal rats. The tubules showing an intense signal in the SHC rat kidneys had remarkably dilated lumens. Although inflammatory portions were found in the SHC rat kidneys, no expression signal of TSC-22 was observed. Using the sense probe, no signal was observed in the SD or SHC rat kidneys.

These results suggest a close association of the high expression of TSC-22 in the glomerular and tubular epithelial cell systems and the onset of renal disease.

### Industrial Applicability

According to the screening method of the present invention, it is possible to screen for a prophylactic or therapeutic agent for a renal disease, diabetes and the like, which is excellently effective and free from side effects.

## Claims

1. A screening method for a prophylactic or therapeutic substance for a disease associated with a protein comprising the same or substantially the same amino acid sequence as the amino acid sequence shown by SEQ ID NO:2 or a salt thereof, which comprises using said protein or a partial peptide thereof or a salt thereof.

2. A screening method for a prophylactic or therapeutic substance for a disease associated with a protein comprising the amino acid sequence shown by SEQ ID NO:2 or a salt thereof, which comprises using said protein or a partial peptide thereof or a salt thereof.

3. The screening method of claim 1, wherein the disease is diabetes or a renal disease.

4. The screening method of claim 1, wherein the disease is diabetic nephropathy.

5. The screening method of claim 1, which comprises cultivating a cell having an ability to produce a protein comprising the same or substantially the same amino acid sequence as the amino acid sequence shown by SEQ ID NO:2 or a partial peptide thereof or a salt thereof in the presence and absence of a test substance, and comparing the amounts of said protein or a partial peptide thereof or a salt thereof produced under the two conditions.

6. A screening kit for a prophylactic or therapeutic substance for a disease associated with a protein comprising the same or substantially the same amino acid sequence as the amino acid sequence shown by SEQ ID NO:2 or a salt thereof, which includes (a) a cell having an ability to produce said protein or a partial peptide thereof or a salt thereof, and (b) a substance selected from the group consisting of an antibody against said protein or a partial peptide thereof or a salt thereof, a polynucleotide to which said protein or a partial peptide thereof or a salt thereof can bind, and a transcription regulatory factor capable of interacting with said protein or a partial peptide thereof or a salt thereof.

7. The screening method of claim 1, which comprises comparing the activities of a protein comprising the same or substantially the same amino acid sequence as the amino acid sequence shown by SEQ ID NO:2 or a partial peptide thereof or a salt thereof in the presence and absence of a test substance.

8. A screening kit for a prophylactic or therapeutic substance for a disease associated with a protein comprising the same or substantially the same amino acid sequence as the amino acid sequence shown by SEQ ID NO:2 or a salt thereof, which includes (a) said protein or a partial peptide thereof or a salt thereof, and (b) a polynucleotide to which said protein or a partial peptide thereof or a salt thereof can bind or a transcription regulatory factor capable of interacting with said protein or a partial peptide thereof or a salt thereof.

9. The screening method of claim 7, which comprises cultivating a cell containing a gene whose expression is controlled by a protein comprising the same or substantially the same amino acid sequence as the amino acid sequence shown by SEQ ID NO:2 or a partial peptide thereof or a salt thereof with said protein or a partial peptide thereof or a salt thereof in the presence and absence of a test substance, and comparing the expressions of said gene under the two conditions.

10. A screening kit for a prophylactic or therapeutic substance for a disease associated with a protein comprising the same or substantially the same amino acid sequence as the amino acid sequence shown by SEQ ID NO:2 or a salt thereof, which includes (a) a cell containing a gene whose expression is controlled by said protein or a partial peptide thereof or a salt thereof, (b) said protein or a partial peptide thereof or a salt thereof, and (c) a polynucleotide capable of hybridizing to said gene under highly stringent conditions.

11. The screening method of claim 7, which comprises cultivating a cell having an ability to produce a protein comprising the same or substantially the same amino acid sequence as the amino acid sequence shown by SEQ ID NO:2 or a partial peptide thereof or a salt thereof in the presence and absence of a test substance, and comparing the activities of said protein or a partial peptide thereof or a salt thereof under the two conditions.

12. A screening kit for a prophylactic or therapeutic substance for a disease associated with a protein comprising the same or substantially the same amino acid sequence as the amino acid sequence shown by SEQ ID NO:2 or a salt thereof, which includes (a) a cell having an ability to produce said protein or a partial peptide thereof or a salt thereof, and (b) a polynucleotide capable of hybridizing to a gene whose expression is controlled by said protein or a partial peptide thereof or a salt thereof under highly stringent conditions.

13. A screening method for a prophylactic or therapeutic substance for a disease associated with a protein comprising the same or substantially the same amino acid sequence as the amino acid sequence shown by SEQ ID NO:2 or a salt thereof, which comprises using a polynucleotide comprising the base sequence that encodes said protein or a partial peptide thereof.

14. A screening method for a prophylactic or therapeutic substance for a disease associated with a protein comprising the amino acid sequence shown by SEQ ID NO:2 or a salt thereof, which comprises using a polynucleotide comprising the base sequence that encodes said protein or a partial peptide thereof.

15. The screening method of claim 14, wherein the polynucleotide comprises the entire or a portion of the base sequence shown by SEQ ID NO:1.

16. The screening method of claim 14, wherein the disease is diabetes or a renal disease.

17. The screening method of claim 14, wherein the disease is diabetic nephropathy.

18. The screening method of claim 14, which comprises cultivating a cell having an ability to produce a protein comprising the same or substantially the same amino acid sequence as the amino acid sequence shown by SEQ ID NO:2 or a partial peptide thereof or a salt thereof in the presence and absence of a test substance, and comparing the amounts of mRNA that encodes said protein or a partial peptide thereof under the two conditions.

19. A screening kit for a prophylactic or therapeutic substance for a disease associated with a protein comprising the same or substantially the same amino acid sequence as the amino acid sequence shown by SEQ ID NO:2 or a salt thereof, which includes (a) a cell having an ability to produce said protein or a partial peptide thereof or a salt thereof, and (b) a polynucleotide capable of hybridizing to mRNA that encodes said protein or a partial peptide thereof under highly stringent conditions.

20. A prophylactic or therapeutic agent for a disease associated with a protein comprising the same or substantially the same amino acid sequence as the amino acid sequence shown by SEQ ID NO:2 or a salt thereof, which contains an antibody against said protein or a partial peptide thereof or a salt thereof.

21. The prophylactic or therapeutic agent of claim 20, wherein the disease is diabetes or a renal disease.

22. The prophylactic or therapeutic agent of claim 20, wherein the disease is diabetic nephropathy.

23. A prophylactic or therapeutic agent for a disease associated with a protein comprising the same or substantially the same amino acid sequence as the amino acid sequence shown by SEQ ID NO:2 or a salt thereof, which contains a polynucleotide having a base sequence complementary to the base sequence that encodes said protein or a partial peptide thereof.

24. The prophylactic or therapeutic agent of claim 23, wherein the disease is diabetes or a renal disease.

25. The prophylactic or therapeutic agent of claim 23, wherein the disease is diabetic nephropathy.

26. A diagnostic reagent for a disease associated with a protein comprising the same or substantially the same amino acid sequence as the amino acid sequence shown by SEQ ID NO:2 or a salt thereof, which contains an antibody against said protein or a partial peptide thereof or a salt thereof.

27. The diagnostic reagent of claim 26, wherein the disease is diabetes or a renal disease.

28. The diagnostic reagent of claim 26, wherein the disease is diabetic nephropathy.

29. A diagnostic reagent for a disease associated with a protein comprising the same or substantially the same amino acid sequence as the amino acid sequence shown by SEQ ID NO:2 or a salt thereof, which contains a polynucleotide comprising the base sequence that encodes said protein or a partial peptide thereof.

30. The diagnostic reagent of claim 29, wherein the disease is diabetes or a renal disease.

31. The diagnostic reagent of claim 29, wherein the disease is diabetic nephropathy.

32. A prophylactic or therapeutic agent for diabetes or a renal disease, which contains a TSC-22 suppressant.

33. The prophylactic or therapeutic agent of claim 32, wherein the renal disease is diabetic nephropathy.

34. A prophylactic or therapeutic method for diabetes or a renal disease in a mammal, which comprises administering a TSC-22 suppressant to said mammal.

35. The method of claim 34, wherein the renal disease is diabetic nephropathy.

36. Use of a TSC-22 suppressant for the production of a prophylactic or therapeutic agent for diabetes or a renal disease.

37. The use of claim 36, wherein the renal disease is diabetic nephropathy.


SEQUENCE LISTING

<110>  Takeda Chemical Industries, Ltd.

<120>  Screening Method

<130>  3121WOOP

<150>  JP 2002-329778
<151>  2002-11-13

<160>  3

<170>  PatentIn version 3.1

<210>  1
<211>  432
<212>  DNA
<213>  Homo sapiens

<220>
<221>  CDS
<222>  (1)..(432)
<223>

<400>  1
atg aaa tcc caa tgg tgt aga cca gtg gcg atg gat cta gga gtt tac      48
Met Lys Ser Gln Trp Cys Arg Pro Val Ala Met Asp Leu Gly Val Tyr
1               5                   10                  15

caa ctg aga cat ttt tca att tct ttc ttg tca tcc ttg ctg ggg act      96
Gln Leu Arg His Phe Ser Ile Ser Phe Leu Ser Ser Leu Leu Gly Thr
            20                  25                  30

gaa aac gct tct gtg aga ctt gat aat agc tcc tct ggt gca agt gtg      144
Glu Asn Ala Ser Val Arg Leu Asp Asn Ser Ser Ser Gly Ala Ser Val
        35                  40                  45

gta gct att gac aac aaa atc gag caa gct atg gat cta gtg aaa agc      192
Val Ala Ile Asp Asn Lys Ile Glu Gln Ala Met Asp Leu Val Lys Ser
    50                  55                  60

cat ttg atg tat gcg gtc aga gaa gaa gtg gag gtc ctc aaa gag caa      240
His Leu Met Tyr Ala Val Arg Glu Glu Val Glu Val Leu Lys Glu Gln
65                  70                  75                  80

atc aaa gaa cta ata gag aaa aat tcc cag ctg gag cag gag aac aat      288
Ile Lys Glu Leu Ile Glu Lys Asn Ser Gln Leu Glu Gln Glu Asn Asn
                85                  90                  95

ctg ctg aag aca ctg gcc agt cct gag cag ctt gcc cag ttt cag gcc      336
Leu Leu Lys Thr Leu Ala Ser Pro Glu Gln Leu Ala Gln Phe Gln Ala

100                 105                 110

cag ctg cag act ggc tcc ccc cct gcc acc acc cag cca cag ggc acc        384
Gln Leu Gln Thr Gly Ser Pro Pro Ala Thr Thr Gln Pro Gln Gly Thr
        115                 120                 125

aca cag ccc ccc gcc cag cca gca tcg cag ggc tca gga cca acc gca        432
Thr Gln Pro Pro Ala Gln Pro Ala Ser Gln Gly Ser Gly Pro Thr Ala
    130                 135                 140

<210>  2
<211>  144
<212>  PRT
<213>  Homo sapiens

<400>  2

Met Lys Ser Gln Trp Cys Arg Pro Val Ala Met Asp Leu Gly Val Tyr
1               5                   10                  15

Gln Leu Arg His Phe Ser Ile Ser Phe Leu Ser Ser Leu Leu Gly Thr
            20                  25                  30

Glu Asn Ala Ser Val Arg Leu Asp Asn Ser Ser Ser Gly Ala Ser Val
        35                  40                  45

Val Ala Ile Asp Asn Lys Ile Glu Gln Ala Met Asp Leu Val Lys Ser
    50                  55                  60

His Leu Met Tyr Ala Val Arg Glu Glu Val Glu Val Leu Lys Glu Gln
65                  70                  75                  80

Ile Lys Glu Leu Ile Glu Lys Asn Ser Gln Leu Glu Gln Glu Asn Asn
                85                  90                  95

Leu Leu Lys Thr Leu Ala Ser Pro Glu Gln Leu Ala Gln Phe Gln Ala
            100                 105                 110

Gln Leu Gln Thr Gly Ser Pro Pro Ala Thr Thr Gln Pro Gln Gly Thr
        115                 120                 125

Thr Gln Pro Pro Ala Gln Pro Ala Ser Gln Gly Ser Gly Pro Thr Ala
    130                 135                 140

<210>  3
<211>  342
<212>  DNA
<213>  Artificial Sequence

<220>
<223>  DNA used as sense probe for in situ hybridization analysis in
       Example 4.

<400>  3
gcctgctatg ccccaacaga actggctgct gctgtctgaa ctgaacagac cgaagagatg      60

tgctagtgag aagccgcctc cagtcaccca tttcattgct gtctgcgaaa gagacgtgag     120

actcacacat gctgttctcg ctttctcccc agtattaagc actcatatgc ttttggcttg     180

aagaaatata ctagttgagt gaattaaagg ttaaacagag agtgagcatg gatgtaccct     240

gtgcaacgtg gcagatgtct gaggaatggt ttgattgacg ctgaggagga gctctgtgcc     300

ttttcaaccc tccccagccg cccactctac tcccaagctc tg                        342